# EUROPEAN PATENT SPECIFICATION

(11) **EP 2 246 831 B1**
(45) Date of publication and mention of the grant of the patent: **06.01.2016**
(21) Application number: 09703655.2
(22) Date of filing: 20.01.2009
(51) Int. Cl.: G08G 1/16

(54) **DANGEROUS DRIVE PREVENTIVE INTENTION JUDGMENT SYSTEM AND DANGEROUS DRIVE PREVENTIVE INTENTION JUDGMENT METHOD**
SYSTEM UND VERFAHREN ZUR BEURTEILUNG GEFÄHRLICHER PRÄVENTIVER FAHRMANÖVERABSICHTEN
SYSTÈME ET PROCÉDÉ D'ÉVALUATION D'INTENTION POUR ÉVITER LA CONDUITE DANGEREUSE

(30) Priority: 22.01.2008 JP 2008011239; 15.12.2008 JP 2008318057
(43) Date of publication of application: 03.11.2010
(73) Proprietor: Advanced Telecommunications Research Institute International, Soraku-gun Kyoto 619-0288 (JP)
(72) Inventor: TADA, Masahiro, Soraku-gun Kyoto 619-0288 (JP); RENGE, Kazumi, Soraku-gun Kyoto 619-0288 (JP); NOMA, Haruo, Soraku-gun Kyoto 619-0288 (JP); TORIYAMA, Tomoji, Soraku-gun Kyoto 619-0288 (JP); NAYA, Futoshi, Soraku-gun Kyoto 619-0288 (JP); KOGURE, Kiyoshi, Soraku-gun Kyoto 619-0288 (JP)
(74) Representative: Taor, Simon Edward William
(86) International application number: PCT/JP2009/051146
(87) International publication number: WO 2009/093726

(56) References cited:
- WO-A1-2007/049596
- JP-A- 6 215 300
- JP-A- 2000 111 568
- JP-A- 2002 042 288
- JP-A- 2002 140 775
- JP-A- 2004 038 489
- JP-A- 2005 209 073
- JP-A- 2005 293 032
- JP-A- 2006 227 905
- JP-A- 2007 051 973
- JP-A- 2007 334 479
- JP-A- 2007 334 479
- JP-T- 2007 524 134
- US-A1- 2006 195 241
- US-A1- 2007 008 151

## Description

### TECHNICAL FIELD

The present invention relates to a dangerous drive preventive intention judging system and a dangerous drive preventive intention judging method. More specifically, the present invention relates to a system and method of judging a dangerous drive preventive intention which are for judging a dangerous drive preventive intention of a driver when a motor vehicle approaches a dangerous place.

### BACKGROUND ART

Recently, the number of causalities of traffic accidents is on a declining trend, but a downward trend in the number of occurrences of the accidents itself is not discerned. According to the traffic accident occurring situation statistics (non-patent document 1: National Police Agency Traffic Bureau, traffic accident occurring situation in 2006, February 23, 2007) of National Police Agency, 26.3% of the number of traffic accidents occurred in 2006 is committed by accidents on sudden encounters. In addition, according to the statistics, 52.0% of the accident on sudden encounters occurs at unsignalized intersections in urban areas. Therefore, it may be said that establishment of technique for prevention of accidents at unsignalized intersections remains a critical issue.

In a conventional traffic accident occurring situation investigation at unsignalized intersections, a right-and-left looking motion of a driver by utilizing a video analysis (including an eye mark recorder) is checked, and vehicle velocity at a time of entering an intersection, and operation amounts of an accelerator pedal and/or a brake pedal are measured. Among them, by checking a line of sight with an eye mark recorder or a video image, it is possible to know to a certain extent whether a driver can predict a danger at an unsignalized intersection.

Furthermore, in a Patent Document 1 (Japanese Patent Application Laid-Open No. 2002-331850 [B60K 28/06, A61B 3/113, 5/18, B60K 28/16, B60R 21/00]), from a direction frequency distribution of the line of sight of the driver detected by an eye mark recorder and operation amounts of an accelerator pedal and/or a brake pedal, a technique of predicting a driving motion intention is disclosed.

On the one hand, a taxicab business world takes various measures to reduce accidents by taxi drivers at the present. A reeducation course of driving aimed at a taxi driver who caused an accident is especially frequently performed. However, the reeducation course is just ex post measures, and the effect of the course is reduced with time. It is conceivable that the taxi driver who is considered to have a high probability of causing an accident is preselected, and is made to take the reeducation course, but as a selecting means therefor, it is merely possible to depend on a self-assessed type questionnaire test, such as a driving aptitude test in reality.

However, the eye mark recorder is so extensive, and it is unreasonable to regularly use it during a daily driving. Furthermore, it is considered that in the light of a dangerous drive preventive intention, there is a major difference between a person who moves the foot to the brake pedal before entering the intersection and prepares for emergency (hereinafter to be referred as "braking stance") even if the brake pedal is not operated, and a person who does not move the foot from the accelerator pedal, but even if only an operation amount of the accelerator pedal and/or the brake pedal is concentrated, it is impossible to judge whether a "braking stance" is taken.

On the other hand, in the self-assessed type questionnaire test for preselection of a subject to take the reeducation course, the dangerous drive preventive intention of the driver cannot be quantitatively measured, and therefore, it is difficult to make a proper judgment.

US 2006/195241 A1 discloses a vehicle information notification device which is able to evaluate the right-and-left safe-condition check state of a driver according to the geometric form of an intersection.

### SUMMARY OF THE INVENTION

Therefore, it is a primary object of the present invention to provide a novel system and method of judging a dangerous drive preventive intention.

Another object of the present invention is to provide a system and method of judging a dangerous drive preventive intention capable of judging a right-and-left checking motion and a braking stance without placing a significant burden on a driver.

Another object of the present invention is to provide a system and method of judging a dangerous drive preventive intention capable of quantitatively measuring a dangerous drive preventive intention of a driver.

The invention is defined in the appended claims.

The present invention employs following features in order to solve the above-described problems. It should be noted that reference numerals and the supplements inside the parentheses show one example of a corresponding relationship with the embodiments described later for easy understanding of the present invention, and do not limit the present invention.

A first embodiment is a system of judging a dangerous drive preventive intention for judging a dangerous drive preventive intention of a driver of a motor vehicle with respect to a dangerous place, and comprises a first movement detector which detects a movement of a head of the driver; a position detector which detects position data of the motor vehicle; a first judger which judges whether or not a visually checking motion is performed on the basis of the movement data detected by the first movement detector when the motor vehicle approaches the dangerous place on the basis of the position data detected by the position detector; and an alarmer which issues an alarm when the first judger judges that a visually checking motion was not performed.

In the first embodiment, a dangerous drive preventive intention judging system (10) judges whether or not a driver performs a visually checking motion in order to judge a dangerous drive preventive intention of a driver of a vehicle with respect to a dangerous place, such as an unsignalized intersection, an accident-prone place, for example. A first movement detector (14) is for detecting a movement of a head of the driver. The first movement detector may be an angular velocity sensor, an acceleration sensor, or the like. In a case that an angular velocity sensor is applied, the angular velocity sensor is attached to a head of the driver, for example, by being attached to a cap (26) of the driver, and detects a rotation of the head of the driver. If the driver turns to a left direction in order to perform a visual check, an angular velocity to the left direction is detected, and if the driver turns to a right direction, an angular velocity to the right direction is detected. Accordingly, by detecting the rotation of the head using the angular velocity sensor, a visually checking motion of the driver can be measured. A position detector (12, 18, S1) detects position data of the motor vehicle. The position detector is a GPS receiver, for example, and detects coordinates (latitude, longitude, altitude) of a current position of the motor vehicle. A first judger (12, S13, S41-S47) judges whether or not a visually checking motion is performed on the basis of the movement data detected by the first movement detector when the motor vehicle approaches the dangerous place on the basis of the position data detected by the position detector. Here, information as to a position of the dangerous place is previously stored in a memory (20) as map data (road data), for example, and on the basis of the map data and the position data, it is determined whether or not the motor vehicle approaches the dangerous place. Furthermore, in a case that an angular velocity sensor is applied as a first movement detector, angular velocity data is detected as movement data, and therefore, it is possible to judge whether or not a visually checking motion is performed on the basis of the angular velocity data. Then, an alarmer (12, 22, S17-S21) issues an alarm when the first judger judges that a visually checking motion was not performed. The alarm is performed by a sound output, an image display, a vibration output, or the like.

According to the first embodiment, the movement of the head of the driver is detected by using the motion detector, so that it is possible to measure a visually checking motion of the driver without placing a burden on the driver, and it is possible to easily judge whether or not a visually checking motion is performed when the vehicle approaches the dangerous place. Then, when a visually checking motion was not performed even if the vehicle approaches the dangerous place, an alarm can be output, thus, it is possible to urge the driver to perform a visually checking motion and heighten a dangerous drive preventive intention of the driver.

A second embodiment is according to the first embodiment, and further comprises a first video image data saver which saves video image data of at least one of an in-vehicle video image and an out-vehicle video image of the motor vehicle when the first judger judges that a visually checking motion was not performed.

In the second embodiment, a shooter (video camera 36a, 36b) capable of shooting at least one of an in-vehicle video image and an out-vehicle video image of the motor vehicle is provided, and a first video image data saver (12, 22) saves video image data of the video shot by the shooter.

According to the embodiment invention, by reproducing video image data saved by the first video image data saver, it is possible to show the driver a situation (video image) in which a necessary visually checking motion was not performed, and therefore, it is possible to more strongly urge the driver to perform a visually checking motion and heighten a dangerous drive preventive intention of the driver.

A third embodiment is according to the first or the second embodiment, and further comprises a first sound data saver which saves sound data of at least an in-vehicle sound of the motor vehicle when the first judger judges that a visually checking motion was not performed.

In the third embodiment a sound capturer (microphone 38) capturing sound data of at least an in-vehicle sound of the motor is provided, and a first sound data saver (12, S22) saves sound data of the sound captured by the capturer.

According to the third embodiment, by producing the sound data saved by the first sound data saver, it is possible to show the relevant driver a situation (sound) when a necessary visually checking motion was not performed, and therefore, it is possible to more strongly urge the driver to perform a visually checking motion and heighten a dangerous drive preventive intention of the driver.

A fourth embodiment is a dangerous drive preventive intention judging system according to the first embodiment, and further comprises: a second movement detector which detects a motion of a right toe of the driver; and a second judger which judges whether or not a braking stance is taken on the basis of movement data detected by the second movement detector when it is determined that the motor vehicle approaches the dangerous place on the basis of the position data detected by the position detector; wherein the alarmer issues an alarm when the second judger judges that a braking stance was not taken.

In the fourth embodiment in order to judge the dangerous drive preventive intention of the driver, it is judged whether or not the driver performs a braking stance. A second movement detector (16) is for detecting a motion of a right toe of the driver. The second movement detector is an angular velocity sensor. In a case that the angular velocity sensor is applied, the angular velocity sensor is attached to the right toe of the driver with which the driver performs accelerating and braking operations, and detects a rotation of the right toe of the driver. If the driver moves the right foot to a side of the brake pedal (32), the angular velocity to the left direction is detected, and if the driver moves the right foot to the side of the accelerator pedal (30), the angular velocity to the right direction is detected. Thus, by detecting a rotation of the right toe by using the angular velocity sensor, it is possible to measure whether the driver moves the right foot to the side of the brake pedal, that is, whether or not a braking stance is taken. A second judger (12, S15, S61-S75) judges whether or not a braking stance is taken on the basis of movement data detected by the second movement detector when it is determined that the motor vehicle approaches the dangerous place. If an angular velocity sensor is applied as a second movement detector, angular velocity data is detected as movement data, and therefore, whether or not a braking stance is taken is judged on the basis of the angular velocity data. Then, an alarmer outputs an alarm when it is judged that a braking stance was not performed as well.

According to the fourth embodiment, the movement of the right toe of the driver is further detected by using the movement detector, and therefore, it is possible to measure a braking stance of the driver without placing a burden on the driver, and it is possible to easily judge whether or not a braking stance is taken when the vehicle approaches the dangerous place. Moreover, when a braking stance is not taken, it is possible to issue an alarm to the driver, so that it is possible to urge the driver to take a braking stance by releasing the right foot from the accelerator and take a braking stance and heighten a dangerous drive preventive intention of the driver.

A fifth embodiment is a dangerous drive preventive intention judging system judging a dangerous drive preventive intention of a driver of a motor vehicle with respect to a dangerous place, comprising: a second movement detector which detects a movement of a right toe of the driver; a position detector which detects position data of the motor vehicle; a second judger which judges whether or not a braking stance is taken on the basis of movement data detected by the second movement detector when it is determined that the motor vehicle approaches the dangerous place on the basis of the position data detected by the position detector; and an alarmer which issues an alarm when the second judger judges that a braking stance was not taken.

In the fifth embodiment, a dangerous drive preventive intention judging system (10) judges whether or not a driver takes a braking stance in order to judge a dangerous drive preventive intention of a driver of a vehicle with respect to a dangerous place, such as an unsignalized intersection, an accident-prone place, for example. A second movement detector (16) is for detecting a movement of a right toe of the driver. The second movement detector may be an angular velocity sensor, an acceleration sensor, or the like. In a case that an angular velocity sensor is applied, the angular velocity sensor is attached to the right toe of the driver with which the driver performs accelerating and braking operations, and detects a rotation of the right toe of the driver. If the driver moves the right foot to a side of the brake pedal (32), the angular velocity to the left direction is detected, and if the driver moves the right foot to the side of the accelerator pedal (30), the angular velocity to the right direction is detected. Thus, by detecting a rotation of the right toe by using the angular velocity sensor, it is possible to measure whether the driver moves the right foot to the side of the brake pedal, that is, whether or not a braking stance is taken. A position detector (12, 18, S1) detects position data of the motor vehicle. The position detector is a GPS receiver, for example, and detects coordinates (latitude, longitude, altitude) of a current position of a motor vehicle. A second judger (12, S15, S61-S75) judges whether or not a braking stance is taken on the basis of movement data detected by the second movement detector when it is determined that the motor vehicle approaches the dangerous place. Here, information as to a position of the dangerous place is previously stored in the memory (20) as map data (road data), for example, and on the basis of the map data and the position data, it is determined whether or not the motor vehicle approaches the dangerous place. If an angular velocity sensor is applied as a second movement detector, angular velocity data is detected as movement data, and therefore, on the basis of the angular velocity data, whether or not a braking stance is taken is judged. An alarmer (12, 22, S17-S21) issues an alarm when the second judger judges that a braking stance was not performed. The alarm is performed by a sound output, an image display, a vibration output or the like.

According to the fifth embodiment, the movement of the right toe of the driver is further detected by using the movement detector, and therefore, it is possible to measure a braking stance of the driver without placing a burden on the driver, and it is possible to easily judge whether or not a braking stance is taken when the vehicle approaches the dangerous place. Moreover, when a braking stance is not taken, it is possible to output an alarm to the driver, so that it is possible to urge the driver to take a braking stance by releasing the right foot from the accelerator and take a braking stance, and heighten a dangerous drive preventive intention of the driver.

A sixth embodiment is according to the fifth embodiment, and further comprises a second video image data saver which saves video image data of at least one of an in-vehicle video image and an out-vehicle video image of the motor vehicle when the second judger judges that a braking stance was not taken.

In the sixth embodiment, a shooter (video camera 36a, 36b) capable of shooting at least one of an in-vehicle video image and an out-vehicle video image of the motor vehicle is provided, and a second video image data saver (12, S22) saves video image data of the video shot by the shooter.

According to the sixth embodiment by reproducing the video image data saved by the second video image data saver, it is possible to show the driver a situation (video) in which a necessary braking stance was not taken, and it is possible to more strongly urge the driver to take a braking stance and heighten a dangerous drive preventive intention of the driver.

A seventh embodiment is according to the fifth or the sixth embodiment, and further comprising a second sound data saver which saves sound data of at least an in-vehicle sound of the motor vehicle when the second judger judges that a braking stance was not taken.

In the seventh embodiment, a sound capturer (microphone 38) capturing sound data of at least an in-vehicle sound of the motor is provided, and a second sound data saver (12, S22) saves sound data of the sound captured by the sound capturer.

According to the seventh embodiment by reproducing the sound data saved by the second sound data saver, it is possible to show the drive a situation (sound) when a necessary braking stance was not taken, and therefore, it is possible to more strongly urge the driver to take a braking stance and heighten a dangerous drive preventive intention of the driver.

An eighth embodiment is a dangerous drive preventive intention judging system judging a dangerous drive preventive intention of a driver of a motor vehicle with respect to a dangerous place, comprising: a first movement detector which detects a movement of a head of the driver; a position detector which detects position data of the motor vehicle; a first judger which determines whether or not a visually checking motion is performed on the basis of movement data detected by the first movement detector when it is determined that the motor vehicle approaches the dangerous place on the basis of the position data detected by the position detector; and a recorder which records a judgment result by the first judger.

In the eighth embodiment a dangerous drive preventive intention judging system (10) comprises a first movement detector, a position detector, and a first judger similar to the above-described first invention in order to judge a dangerous drive preventive intention of a driver with respect to a dangerous place. That is, the dangerous drive preventive system can judge whether or not the driver performs a visually checking motion when the motor vehicle approaches the dangerous place. In addition, the dangerous drive preventive intention judging system comprises a recorder (12, 24, S23), and the recorder records a judgment result by the first judger.

According to the eighth embodiment, similar to the first embodiment, the movement of the head of the driver is detected by using the motion detector, so that it is possible to measure a visually checking motion of the driver without placing a burden on the driver, and it is possible to easily judge whether or not a visually checking motion is performed when the vehicle approaches the dangerous place. Moreover, a judgment result of the visually checking motion with respect to the dangerous place can be recorded. Thus, it is possible to quantitatively measure the dangerous drive preventive intention of the driver and evaluate a dangerous drive preventive intention of the driver.

A ninth embodiment is a dangerous drive preventive intention judging system according to the eighth embodiment, further comprising: a second movement detector which detects a movement of a right toe of the driver; and a second judger judges whether or not a braking stance is taken on the basis of movement data detected by the second movement detector when it is determined that the motor vehicle approaches the dangerous place on the basis of the position data detected by the position detector; wherein the recorder further records a judgment result by the second judger.

In the ninth embodiment a dangerous drive preventive intention judging system further comprising a second movement detector and second judger similar to the above-described second invention. That is, the dangerous drive preventive intention judging system can further judge whether or not a braking stance is taken when the motor vehicle approaches the dangerous place. The recorder further records a judgment result by the second judger.

According to the ninth embodiment, the movement of the right toe of the driver is detected by using the movement detector, and therefore, it is possible to further measure a braking stance of the driver without placing a burden on the driver, and it is possible to easily judge whether or not a braking stance is taken when the vehicle aproaches the dangerous place. Then, judgment results of the visually checking motion and the braking stance of the driver with respect to the dangerous place can be recorded. Thus, it is possible to quantitatively measure the dangerous drive preventive intention based on both of the visually checking motion and the braking stance, and evaluate the dangerous drive preventive intention of the driver.

A tenth embodiment is a dangerous drive preventive intention judging system judging a dangerous drive preventive intention of a driver of a motor vehicle with respect to a dangerous place, comprising: a second movement detector which detects a movement of a right toe of the driver; a position detector which detects position data of the motor vehicle; a second judger which judges whether or not a braking stance is taken on the basis of movement data detected by the second movement detector when it is determined that the motor vehicle approaches the dangerous place on the basis of the position data detected by the position detector; and a recorder which records a judgment result of the second judger.

In the tenth embodiment, a dangerous drive preventive intention judging system (10) comprises a second movement detector, a position detector, and a second judger similar to the above-described fourth embodiment That is, the dangerous drive preventive intention judging system can judge whether or not the driver takes a braking stance when the motor vehicle approaches the dangerous place. In addition, the tenth invention comprises a recorder (12, 24, S23), and the recorder records a judgment result by the second judger.

According to the tenth embodiment, similar to the above-described fourth embodiment, the movement of the right toe of the driver is further detected by using the movement detector, and therefore, it is possible to measure a braking stance of the driver without placing a burden on the driver, and it is possible to easily judge whether or not a braking stance is taken when the vehicle approaches the dangerous place. Then, it is possible to record a determination result of the braking stance with respect to the dangerous place. Thus, it is possible to quantitatively measure a dangerous drive preventive intention of the driver and evaluate the dangerous drive preventive intention of the driver.

An eleventh embodiment is dangerous drive preventive intention judging system judging a dangerous drive preventive intention of a driver of a motor vehicle with respect to a dangerous place, comprising: a memory which stores movement data detected and recorded by a first movement detector detecting a movement of a head of the driver and position data of the motor vehicle detected and recorded by a position detecting device; a first judger which judges whether or not a visually checking motion is performed when a position of the motor vehicle approaches the dangerous place on the basis of the movement data by the first movement detector and the position data of the motor vehicle; and a result outputter which outputs a judgment result by the first judger.

In the eleventh embodiment in order to judge a dangerous drive preventive intention of a driver of a vehicle with respect to a dangerous place, such as an unsignalized intersection, an accident-prone place, for example, a dangerous drive preventive intention judging system (10) performs an ex post analysis on the data recorded during driving and judges whether or not the driver performs a visually checking motion. A memory (12, 24) stores recording data including movement data of the head of the driver and position data of the motor vehicle. The movement data of the head is data detected and recorded by a first movement detector (14) detecting a movement of the head of the driver. The first movement detector may be an angular velocity sensor, an acceleration sensor or the like. If the angular velocity sensor is applied, a rotation of the head of the driver during driving is detected by the angular velocity sensor, and angular velocity data is recorded as movement data, and thus, in this memory, the angular velocity data of the head is stored. Furthermore, the position data of the motor vehicle is data detected and recorded by a position detecting device (18), such as a GPS receiver, for example. The first judger (12, S109) judges whether or not a visually checking motion is performed when a position of the motor vehicle approaches the dangerous place on the basis of the movement data by the first movement detector and the position data of the motor vehicle. Here, information as to a position of the dangerous place is previously stored in a memory (12, 20) as map data (road data), for example, and it is determined whether or not the motor vehicle approaches the dangerous place on the basis of the map data and the position data. Furthermore, by analyzing the movement data of the head detected when the motor vehicle approaches the dangerous place, the visually checking motion of the driver can be judged. A result outputter (12, S121) outputs a judgment result by the first judger. For example, the judgment result data may be output by a file, may be displayed on the display, or an evaluation of the judgment result may be output.

According to the eleventh embodiment, it is possible to perform a visually checking motion with respect to the dangerous place ex post facto on the basis of the recorded movement data of the head, the recorded position data of the motor vehicle, etc., and it is possible to quantitatively measure and evaluate the dangerous drive preventive intention of the driver.

A twelfth embodiment is a dangerous drive preventive intention judging system according to the eleventh embodiment wherein the memory further stores movement data detected and stored by a second movement detector detecting a movement of a right toe of the driver, further comprising: a second judger which judges whether or not a braking stance is taken when a position of the motor vehicle approaches the dangerous place on the basis of the movement data by the second movement detector and position data of the motor vehicle, and the result outputter outputs judgment results by the first judger and the second judger.

In the twelfth embodiment, the memory further stores movement data of the right toe of the driver. The movement data of the right toe is data detected and stored by a second movement detector detecting a movement of a right toe of the driver. The second movement detector may be an angular velocity sensor, an acceleration sensor or the like. In a case that the angular velocity sensor is applied, a rotation of the head of the driver during driving is detected by the angular velocity sensor, and angular velocity data is recorded as movement data, and thus, in the memory, the angular velocity data of the right toe is stored. A second judger (12, S113) judges whether or not a braking stance is taken when a position of the motor vehicle approaches the dangerous place on the basis of the movement data of the right toe and position data of the motor vehicle. By analyzing the movement data of the right toe, it is possible to judge whether or not the driver takes a braking stance when the motor vehicle approaches the dangerous place. Then, the result outputter outputs judgment results by the first judger and the second judger.

According to the twelfth embodiment, it is possible to judge a visually checking motion and a braking stance with respect to the dangerous place ex post facto on the basis of the recorded movement data of the head and the right toe of the driver, the position data of the motor vehicle, etc., and it is possible to quantitatively measure and evaluate the dangerous drive preventive intention of the driver on the basis of the visually checking motion and the braking stance by the driver.

A thirteenth embodiment is a dangerous drive preventive intention judging system judging dangerous drive preventive intention of a driver of a motor vehicle with respect to a dangerous place, comprising: a memory which stores movement data detected and recorded by a second movement detector detecting a movement of a right toe of the driver, and position data of the motor vehicle detected and recorded by a position detecting device; a second judger which judges a braking stance is taken when a position of the motor vehicle approaches the dangerous place on the basis of the movement data by the second movement detector and the position data of the motor vehicle; and a result outputter which outputs a judgment result by the second judger.

In the thirteenth embodiment, in order to judge a dangerous drive preventive intention of a driver of a vehicle with respect to a dangerous place, such as an unsignalized intersection, an accident-prone place, for example, a dangerous drive preventive intention judging system (10) performs an ex post analysis on the data recorded during driving and judges whether or not the driver performs a visually checking motion. A memory (12, 24) stores recording data including movement data of the right toe of the driver, and position data of the motor vehicle. The movement data of the right toe is data detected and recorded by a second movement detector (16) detecting a movement of the right toe of the driver. The second movement detector may be an angular velocity sensor, an acceleration sensor or the like. Furthermore, the position data of the motor vehicle is detected and recorded by a position detecting device (18), such as a GPS receiver, for example. A second judger (12, S113) judges whether or not a braking stance is taken when a position of the motor vehicle approaches the dangerous place on the basis of the movement data of the right toe and the position data of the motor vehicle. Here, information as to a position of the dangerous place is previously stored in a memory (12, 20) as map data (road data), for example, and on the basis of the map data and the position data, it is determined whether or not the motor vehicle approaches the dangerous place. Then, by analyzing the movement data of the right toe, whether or not a braking stance is taken when the motor vehicle approaches the dangerous place can be judged. A result outputter (12, S121) outputs a judgment result by the second judger. For example, the judgment result data may be output by a file, may be displayed on the display, or an evaluation of the judgment result may be output.

According to the thirteenth embodiment, it is possible judge a braking stance with respect to the dangerous place ex post facto on the basis of the recorded movement data of the right toe, the recorded position data of the motor vehicle, etc., and it is possible to quantitatively measure and evaluate the dangerous drive preventive intention of the driver.

A fourteenth embodiment is a dangerous drive preventive intention judging system according to any one of the first to thirteenth embodiments, wherein the movement detector is an angular velocity sensor.

In the fourteenth embodiment, an angular velocity sensor is applied as a movement detector, and by the angular velocity sensor, the movements of the head and the right toe of the driver are detected. Accordingly, it is possible to measure a visually checking motion and a braking stance of the driver without placing a burden on the driver, and it is possible to easily judge whether a visually checking motion is performed and a braking stance is taken when the vehicle approaches the dangerous place.

A fifteenth embodiment is a dangerous drive preventive intention judging method judging a dangerous drive preventive intention of a driver of a motor vehicle with respect to a dangerous place, including steps of: (a) recording movement data detected by a first movement detector detecting a movement of a head of the driver; and (b) judging whether or not a visually checking motion is performed when the motor vehicle approaches the dangerous place on the basis of the movement data by the first movement detector.

In the fifteenth embodiment the movement data of the first movement detector detecting a movement of the head of the driver is recorded, and on the basis of the movement data, the visually checking motion is judged, and therefore, it is possible to easily judge the visually checking motion without placing a burden on the driver.

A sixteenth embodiment is dangerous drive preventive intention judging method judging a dangerous drive preventive intention of a driver of a motor vehicle with respect to a dangerous place, including steps of: (a) recording movement data detected by a second movement detector detecting a movement of a right toe of the driver; and (b) judging whether or not a braking stance is taken when the motor vehicle approaches the dangerous place on the basis of the movement data by the second movement detector.

According to the sixteenth embodiment the movement data of the second movement detector detecting the movement of the right toe of the driver is recorded, and on the basis of the movement data, a braking stance is judged, and therefore, it is possible to easily judge a braking stance without placing a burden on the driver.

A seventeenth embodiment is a dangerous drive preventive intention judging method according to any one of the fifteenth to sixteenth embodiments wherein the movement detector is an angular velocity sensor.

In the seventeenth embodiment , as a movement detector, the angular velocity sensor is applied, and therefore, it is possible to measure a visually checking motion and a braking stance of the driver without placing a burden on the driver.

An eighteenth embodiment is a dangerous drive preventive intention judging system judging a dangerous drive preventive intention of a driver of a motor vehicle with respect to a dangerous place, comprising: a motion detector which detects a motion of the driver and outputs motion data; a position detector which detects position data of the motor vehicle; a judger which judges whether or not a danger preventing motion is performed on the basis of the motion data by the motion detector when it is determined that the motor vehicle approaches the dangerous place on the basis of the position data detected by the position detector; and a video image data storage which stores video image data of at least one of an in-vehicle video image and an out-vehicle video image of the motor vehicle when the judger judges that a danger preventing motion was not performed.

In the eighteenth embodiment, a dangerous drive preventive intention judging system (10) judges whether or not a driver performs danger averting or preventing motion in order to judge a dangerous drive preventive intention of a driver of a vehicle with respect to a dangerous place, such as an unsignalized intersection, an accident-prone place, for example. A motion detector (14, 16) detects a motion of the driver by detecting a movement of the head of the driver and a movement of the right foot of the driver. The motion detector may be an angular velocity sensor, an acceleration sensor, or the like.

In a case that the angular velocity sensor is applied to detect the movement of the head of the driver as a motion of the driver, the angular velocity sensor is attached to the head of the driver by being attached to a cap (26) of the driver and detects a rotation of the head of the driver. If the driver turns to a left direction in order to perform a visual check, a angular velocity to the left direction is detected, and if the driver turns to a right direction, a angular velocity to the right direction is detected. Accordingly, by detecting a rotation of the head by utilizing the angular velocity sensor, it is possible to detect the motion of the driver.

In a case that the angular velocity sensor is applied to detect the movement of the right foot of the driver as a motion of the driver, the angular velocity sensor is attached to the right toe of the driver with which the driver performs accelerating and braking operations, and detects a rotation of the right toe of the driver. If the driver moves the right foot to a side of the brake pedal (32), the angular velocity to the left direction is detected, and if the driver moves the right foot to the side of the accelerator pedal (30), the angular velocity to the right direction is detected. Thus, by detecting a rotation of the right toe by utilizing the angular velocity sensor, it is possible to measure whether or not the driver moves the right foot to the side of the brake pedal, that is, whether or not a braking stance is taken.

A position detector (12, 18, S1) detects position data of the motor vehicle. The position detector is a GPS receiver, for example, and detects coordinates (latitude, longitude, altitude) of a current position of the motor vehicle. A judger (12, S13, S41-S47:12, S15, S61-S75) determines whether or not a danger prevention or an averting motion is performed on the basis of the motion data detected by the motion detector when it is determined that the motor vehicle approaches the dangerous place on the basis of the position data. Here, information as to a position of the dangerous place is previously stored in a memory (20) as map data (road data), for example, and on the basis of the map data and the position data, it is determined whether or not the motor vehicle approaches the dangerous place.

A shooter (video camera 36a, 36b) capable of shooting at least one of an in-vehicle video image and an out-vehicle video image of the motor vehicle is provided, and if the judger judges that a danger prevention or an averting motion was not performed, a video image data storage(12, S22) stores video image data of the video shot by the shooter.

According to the eighteenth embodiment, by reproducing the video image data stored by the video image data storage, it is possible to show the driver a situation (video) in which a necessary danger preventing (averting) motion was not performed, and it is possible to more strongly urge the driver to perform a danger preventing (averting) motion and heighten a dangerous drive preventive intention of the driver.

A nineteenth embodiment is according to the eighteenth embodiment, further comprising an alarmer which issues an alarm when the judger judges that a danger preventing motion was not performed.

In the nineteenth embodiment, an alarmer (12, 22, S17-S21) outputs an alarm when the judger judges that a danger preventing motion was not performed. The alarm is performed by a sound output, an image display, a vibration output, or the like.

According to the nineteenth embodiment, even if the vehicle approaches the dangerous place, a danger prevention or an averting motion is not performed, an alarm can be generated, and therefore, it is possible to urge the driver to perform a danger prevention or an averting motion and heighten the dangerous drive preventive intention of the driver.

A twentieth embodiment is a dangerous drive preventive intention judging system judging a dangerous drive preventive intention of a driver of a motor vehicle with respect to a dangerous place, further comprising: a motion detector which detects a motion of the driver and outputs motion data; a position detector which detects position data of the motor vehicle; a judger which judges whether or not a danger preventing motion is performed on the basis of the motion data by the motion detector when it is determined that the motor vehicle approaches the dangerous place on the basis of the position data detected by the position detector; and an alarmer which issues an alarm when the judger judges that a danger preventing motion was not performed.

In the twentieth embodiment a dangerous drive preventive intention judging system (10) judges whether or not a driver performs danger averting or preventing motion in order to judge a dangerous drive preventive intention of a driver of a vehicle with respect to a dangerous place, such as an unsignalized intersection, an accident-prone place, for example. A motion detector (14, 16) detects a motion of the driver by detecting a movement of the head of the driver and a movement of the right foot of the driver. The motion detector may be an angular velocity sensor, an acceleration sensor, or the like.

In a case that the angular velocity sensor is applied to detect a movement of the head of the driver as a motion of the driver, the angular velocity sensor is attached to the head of the driver by being attached to a cap (26) of the driver and detects a rotation of the head of the driver. If the driver turns to a left direction in order to perform a visual check, an angular velocity to the left direction is detected, and if the driver turns to a right direction, a angular velocity to the right direction is detected. Accordingly, by detecting a rotation of the head by utilizing the angular velocity sensor, it is possible to detect a motion of the driver.

In a case that the angular velocity sensor is applied to detect a movement of the right foot of the driver as a motion of the driver, the angular velocity sensor is attached to the right toe of the driver with which the driver performs accelerating and braking operations, and detects a rotation of the right toe of the driver. If the driver moves the right foot to a side of the brake pedal (32), the angular velocity to the left direction is detected, and if the driver moves the right foot to the side of the accelerator pedal (30), the angular velocity to the right direction is detected. Thus, by detecting a rotation of the right toe by utilizing the angular velocity sensor, it is possible to measure whether or not the driver moves the right foot to the side of the brake pedal, that is, whether or not a braking stance is taken.

A position detector (12, 18, S1) detects position data of the motor vehicle. The position detector is a GPS receiver, for example, and detects coordinates (latitude, longitude, altitude) of a current position of the motor vehicle. A judger (12, S13, S41-S47:12, S15, S61-S75) judges whether or not a danger prevention or an averting motion is performed on the basis of the motion data detected by the motion detector when it is determined that the motor vehicle approaches the dangerous place on the basis of the position data. Here, information as to a position of the dangerous place is previously stored in a memory (20) as map data (road data), for example, and it is determined whether or not the motor vehicle approaches the dangerous place on the basis of the map data and the position data.

When the judger judges that a danger preventing motion was not performed, an alarmer (12, 22, S17-S21) outputs an alarm. The alarm is performed by a sound output, an image display, a vibration output, or the like.

According to the twentieth embodiment, a motion of the driver is detected by utilizing the motion detector, and therefore, it is possible to determine whether or not a danger prevention or an averting motion is performed when the motor vehicle approaches the dangerous place. Then, even if the vehicle approaches the dangerous place, a danger prevention or an averting motion was not performed, an alarm can be generated, and therefore, it is possible to urge the driver to perform a danger prevention or an averting motion, and heighten the dangerous drive preventive intention of the driver.

According to the present invention, by utilizing the motion detector, a motion of the driver is detected, and therefore, it is possible to judge whether or not a danger prevention or an averting motion is performed when the vehicle approaches the dangerous place. Then, even if the vehicle approaches the dangerous place, a danger prevention or an averting motion was not performed, an alarm can be generated, and therefore, it is possible to urge the driver to perform a danger prevention or an averting motion, and heighten the dangerous drive preventive intention of the driver.

For example, if a movement of the head of the driver is detected by utilizing a movement detector, it is possible to easily judge whether or not a visually checking motion was performed when the vehicle approaches the dangerous place.

Moreover, if a movement of the right toe of the driver is detected by utilizing the movement detector, it is possible to easily judge whether or not a braking stance was taken when the vehicle approaches the dangerous place.

Additionally, if a visually checking motion and a braking stance are judge by detecting the movement data of the head and the right foot, it is possible to quantitatively measure a dangerous driving preventing ability and intention of the driver.

The above described objects and other objects, features, aspects and advantages of the present invention will become more apparent from the following detailed description of the present invention when taken in conjunction with the accompanying drawings.

### BRIEF DESCRIPTION OF THE DRAWINGS

Figure 1 is a block diagram showing a configuration of a dangerous drive preventive intention judging system of one embodiment of the present invention.
Figure 2 is an illustrative view showing one example of an angular velocity sensor attached to a cap of a driver.
Figure 3 is an illustrative view showing one example of an angular velocity sensor attached to a right toe of the driver.
Figure 4 is an illustrative view showing a situation that the right foot of the driver is moved to a side of the brake pedal.
Figure 5 is an illustrative view showing one example of road data included in map data.
Figure 6 is a graph showing one example of angular velocity data measured when a driver of safe-driving drives carefully.
Figure 7 is a graph showing one example of angular velocity data measured when a driver of unsafe driving drives carelessly.
Figure 8 is a flowchart showing one example of an operation of the dangerous drive preventive intention judging system.
Figure 9 is an illustrative view showing a motion judging area set to a dangerous place.
Figure 10 is a flowchart showing one example of an operation of visual check judging processing in Figure 8.
Figure 11 is a flowchart showing one example of an operation of braking stance judging processing in Figure 8.
Figure 12 is a flowchart showing one example of an operation of another embodiment.
Figure 13 is an illustrative view showing a setting example of a video camera utilized in another embodiment of the present invention.
Figure 14 is an illustrative view showing one example of an out-vehicle video image shot by a video camera of the embodiment.
Figure 15 is an illustrative view showing one example of an in-vehicle video image shot by the video camera of the embodiment.
Figure 16 is a block diagram showing a configuration of the embodiment.
Figure 17 is a flowchart showing one example of an operation of the embodiment.
Figure 18 is an illustrative view showing one example of identifying symbols given to time information of video images in the embodiment.
Figure 19 is a graph showing one example of acceleration data measured in a case that the acceleration sensor is attached to the right toe.

### BEST MODE FOR PRACTICING THE INVENTION

### <First Embodiment>

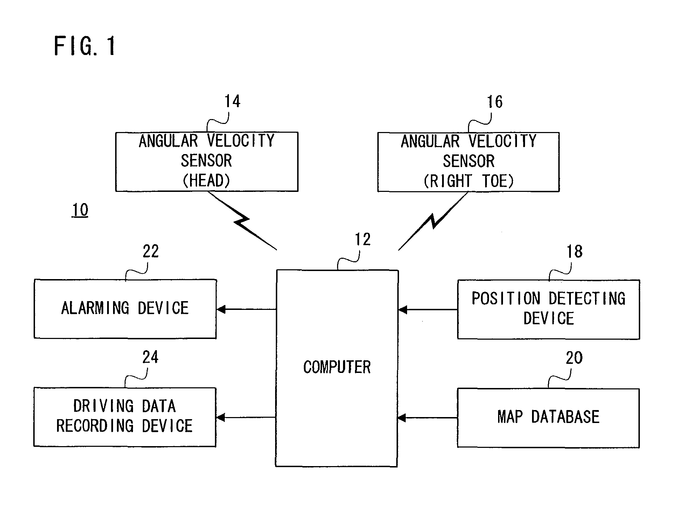

Referring to Figure 1, a dangerous drive preventive intention judging system (hereinafter simply referred to as "system") 10 according to this embodiment is for judging a dangerous drive preventive intention of a driver by judging whether or not the driver performs right and left visual checks and takes a braking stance when a motor vehicle approaches a dangerous place, and includes a computer 12, angular velocity sensors 14, 16, a position detecting device 18, a map database 20, an alarming device 22 and a driving data recording device 24.

The computer 12 is for executing an overall control of the system 10, and is a personal computer, a portable information terminal, etc. but may be a computer of an electronic control unit of the motor vehicle or a computer of a car navigation system.

The computer 12 is, although illustration is omitted, provided with a CPU, a ROM, a RAM, a communication device, etc. The ROM previously stores a control program and data. The RAM is used as a work memory and a buffer memory, and temporarily stores generated data and acquired data, etc. The communication device transmits and receives data with respective devices such as angular velocity sensors 14, 16, etc. to be connected to this computer 12.

In this embodiment, the angular velocity sensor 14 is applied for detecting a movement of a head of the driver, and the angular velocity sensor 16 is applied for detecting a movement of a right toe of the driver. As described later, the angular velocity sensor 14 is for detecting a right and left visually checking motion of the driver, and the angular velocity sensor 16 is for detecting a braking stance of the driver, and therefore, as angular velocity sensors 14 and 16, one capable of detecting at least one-axis angular velocity may be used. Accordingly, a one-axis angular velocity sensor, a two-axis angular velocity sensor or a three-axis angular velocity sensor can be used. Furthermore, as angular velocity sensors 14 and 16, the same kind of sensors may be used. Each of the angular velocity sensors 14, 16 detects an angular velocity about a predetermined axis at a predetermined cycle. Furthermore, each of the angular velocity sensors 14, 16 has a communication function, and transmits the detected angular velocity data to the computer 12 for each predetermined time interval or at a predetermined timing. Furthermore, in this embodiment, each of the angular velocity sensors 14, 16 transmits and receives data with the computer 12 by a short distance wireless communication such as a Bluetooth (registered trademark). Additionally, in another embodiment, each of the angular velocity sensors 14, 16 may be connected to the computer 12 by wire, but may be preferably connected in a wireless manner so as not to prevent the driver from driving the motor vehicle.

The angular velocity sensor 14 is attached to the head of the driver in order to detect a visually checking motion by the driver. When the driver performs a right and left visually checking motion, not only the eyeballs but also the head are generally moved more or less. Thus, by measuring the movement of the head, changes in a gazing direction can be estimated without placing a significant burden on the driver.

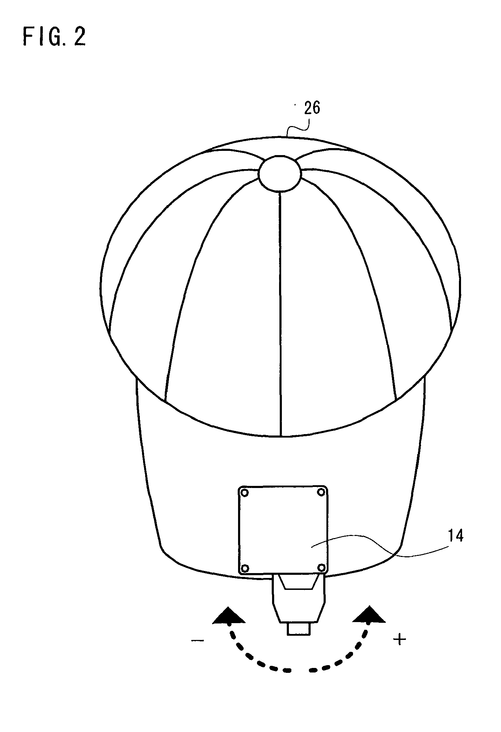

Additionally, for the purpose of implementing easy attachment to the head, a cap 26 is used as shown in Figure 2 in this embodiment, and the angular velocity sensor 14 is attached to a brim of the cap 26, for example. Figure 2 shows a situation that the cap 26 is viewed from above. The angular velocity sensor 14 is attached to the cap 26 so as to detect an angular velocity in correspondence with a rotation about at least an axis of a vertical direction. If the driver puts on the cap 26 attached with the angular velocity sensor 14, angular velocity data in correspondence with a rotation (turn) of the head of the driver in a horizontal direction can be measured, that is, a rotation of the head of the driver. That is, a visually checking motion can be detected and measured. As to a detecting direction of the angular velocity about the axis in the vertical direction of the angular velocity sensor 14, a counterclockwise rotation is set to a positive, and a clockwise rotation is set to a negative direction in Figure 2. If the driver who puts on the cap 26 turns to the left, positive angular velocity data is detected, and if the driver turns to the right, the negative angular velocity data is detected. Thus, by analyzing the velocity data of the angular velocity sensor 14, the computer 12 judges whether or not the driver turns his or her head right or left, that is, whether or not a right or left visually checking motion is performed.

Here, the angular velocity sensor 14 may be attached to the head by other methods, such as a hair band, a hair pin, a bandanna, a head band, etc. In addition, except for direct attachment of the angular velocity sensor 14 to the head, it may be attached by using means, such as pierced earrings, earrings, glasses, a mask, a nose ring, etc.

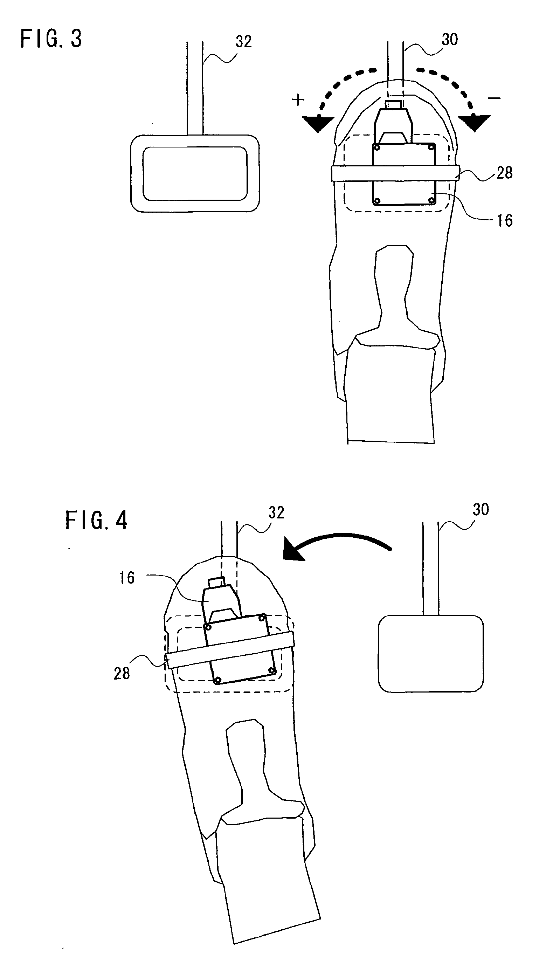

Alternatively, the angular velocity sensor 16 is attached to the right toe of the driver for detecting a braking stance of the driver as shown in Figure 3. The braking stance is considered to be an index to know whether or not the driver predicts a danger, whether or not a preparation in a case of an emergency is made, and therefore, by attaching the angular velocity sensor 16 to the right toe, the dangerous drive preventive intention is measurable.

The angular velocity sensor 16 is attached to the right toe of the driver by using a band 28, etc. Here, the angular velocity sensor 16 may be attached to the right toe by other methods, and the angular velocity sensor 16 may be contained at a part of a toe of driving shoes. In addition, it is conceivable that this may be put on the foot by using means, such as an anklet (leg ring put on the anklet), socks, nail (artificial nail), etc.

In addition, Figure 3 shows the angular velocity sensor 16 when viewed from the driver. The angular velocity sensor 16 is attached to the right toe of the driver so as to detect an angular velocity in correspondence with a rotation about at least an axis of a vertical direction. As to a detecting direction of the angular velocity about the axis in the vertical direction of the angular velocity sensor 16, a counterclockwise rotation is set to a positive, and a clockwise rotation is set to a negative direction in Figure 3.

During driving of the motor vehicle, the right foot of the driver is put on an accelerator pedal 30 as shown in Figure 3. In a case that a braking stance is taken or deceleration is made, the right foot of the driver is moved to a side of a brake pedal 32 as shown in Figure 4, and in a case that the velocity is maintained or acceleration is made thereafter, the right foot is returned to a side of the accelerator pedal 30 as shown in Figure 3. The movement between the accelerator pedal 30 and the brake pedal 32 causes a rotation in a- horizontal direction, and therefore, by detecting a rotation of the right foot by the angular velocity sensor 16, it is possible to judge whether the driver takes a braking stance.

As described above, the movement of the head, that is, the motion of the driver can be detected by the angular velocity sensor 14, and the movement of the right foot, that is, the motion of the driver can be detected by the angular velocity sensor 16. Accordingly, these angular velocity sensors 14 and 16 function as motion detectors for detecting motions of the driver. Then, the angular velocity data from the angular velocity sensors 14 and 16 can be said to be so-called motion data representing a motion of human.

Returning to Figure 1, the position detecting device 18 is for detecting a current position of the motor vehicle. In this embodiment, the position detecting device 18 includes a GPS receiver, and the GPS receiver receives a signal from a GPS satellite to calculate coordinates (latitude, longitude, altitude) of the current position, and outputs position data including the coordinates of the current position to the computer 12.

Here, the position detecting device 18 may include an acceleration sensor, a speed sensor, an angular velocity sensor, etc. mounted on the motor vehicle for estimating a position in a place where a signal from the GPS satellite is not received. Furthermore, as a position detecting device 18 and the map database 20 described later, a car navigation system mounted on the motor vehicle may be used.

The map database 20 previously stores map data. As one example, this may be an HDD including a magnetic disk stored with map data, and an optical disk drive including an optical disk stored with map data, etc. The computer 12 can determine whether or not the current position approaches a dangerous place on the basis of the position data acquired from the position detecting device 18 and the map data acquired from the map database 20.

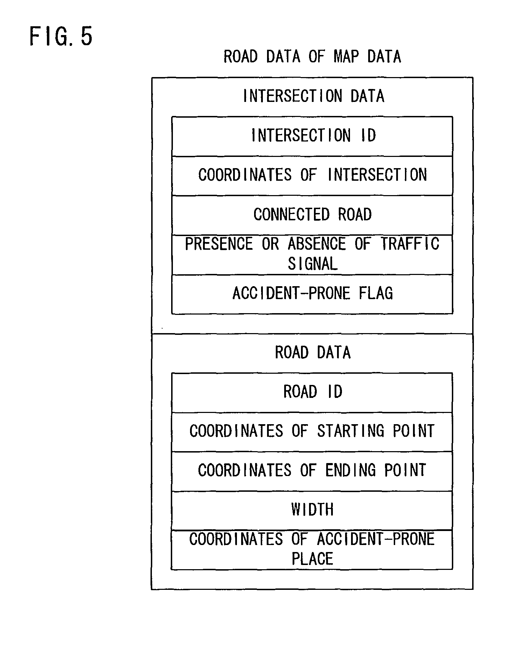

The map data stored in the map database 20 includes road data indicating information in relation to roads, building data indicating information in relation to buildings, etc. Figure 5 shows one example of the road data. The road data includes intersection data indicating information in relation to an intersection and road data indicating information in relation to the road (connection) between the intersections.

The intersection data includes information of a plurality of intersections, and information of each intersection is stored by being brought into correspondence with identifying information (intersection ID) of the intersection. The intersection data includes coordinates of an intersection, a connected road, the presence or absence of a traffic signal, an accident-prone flag, etc.

The coordinates of an intersection include a latitude, a longitude and an altitude of the intersection. As a connected road, a road ID of the road connected to the relevant intersection is registered. The computer 12 can specify a direction of the road connected to the intersection on the basis of the information of the connected road and sets the direction of the connected road as a visually checking direction if the intersection is a dangerous place. Furthermore, if none is registered as information of the presence or absence of a traffic signal, that is, in a case of an unsignalized intersection, the intersection is judged as a dangerous place. In addition, the accident-prone flag is turned on as to the intersection in need of a dangerous drive preventive intention where accidents frequently occur in the past. Accordingly, if information indicating "ON" is registered as information of the accident-prone flag, the intersection is determined as a dangerous place.

The road data includes information of a plurality of roads, and information of each road is stored by being brought into correspondence with identifying information (road ID) of the road. The road data includes coordinates of a starting point, coordinates of an ending point, a width, coordinates of an accident-prone place, etc.

The coordinates of the starting point and the ending point are coordinates (latitude, longitude, altitude) of both ends of the road. From the coordinates of the starting point and the ending point, a direction of the road can be calculated. Furthermore, because the width is registered together with the coordinates of the starting point and the ending point, the computer 12 can specify on which road the motor vehicle drives, and can thus determine whether or not there is a dangerous place down the road. Moreover, the coordinates (latitude, longitude, altitude) of an accident-prone place is registered in a case that a place in need of a dangerous drive preventive intention, such as a place where accidents frequently occur in the past exists on the road. This accident-prone place is also determined as a dangerous place.

Returning to Figure 1, the alarming device 22 is an output device for issuing an alarm (warning) when it is determined that the driver did not perform a necessary motion when approaching the dangerous place. For example, a sound outputting device, a display, a vibration outputting device, etc. may be provided. Here, the alarming device 22 is provided in a case that the system 10 is configured so as to perform judgment for a dangerous drive preventive intention in real time and output an alarm in accordance with the determination result.

In a case of the sound outputting device, a warning sound, a warning voice, etc. are output from the speaker on the basis of sound data, sound data, etc. stored in the computer 12 in advance. The warning sound, the warning voice, etc. may be made different depending on the motion which was not performed by the driver. For example, in a case that a visually checking motion was not performed, a voice instructing the driver to perform a visual check in a direction in which a visual check is required (left, right, or both ways) may be output, or in a case that a braking stance is not taken, a voice instructing the driver to release a foot from the accelerator to take a braking stance may be output.

In a case of the display, a warning image is displayed on the basis of the image data previously stored in the computer 12. The warning image is also made different depending on the motion which was not performed by the driver similar to the above-described sound output case.

Here, as a sound outputting device and a display, a car navigation system mounted on the motor vehicle may be utilized.

The vibration outputting device is contained in a seat or attached to the driver for the purpose of alarming the driver by the vibrations. The vibration outputting device includes a vibrating motor, etc. and outputs vibrations in response to an instruction from the computer 12. Here, a vibrating pattern and intensity may be made different depending on the motion which was not performed by the driver.

The alarming device 22 outputs an alarm when the driver did not perform a motion for a dangerous driving prevention even if the motor vehicle approaches a dangerous place, and therefore, it is possible to urge the drive to perform a necessary motion and heighten a dangerous drive preventive intention of the driver.

The driving data recording device 24 is a storing device for recording various data acquired during driving. This may be a flash memory incorporated in the computer 12, an HDD internal or external to the computer 12, a storage medium connected to the computer 12 like a memory card, or the like. The computer 12 records position data detected by the position detecting device 18, angular velocity data detected by the angular velocity sensors 14 and 16, etc. in the driving data recording device 24. The position data, the angular velocity data, etc. are stored by being brought into correspondence with the acquired time. Alternatively, the position data and the angular velocity data may be stored by being associated with each other. Furthermore, in a case that a dangerous drive preventive intention is judged in real time, data indicating judgment results of a visual check and a braking stance with respect to a dangerous place may be recorded in the driving data recording device 24.

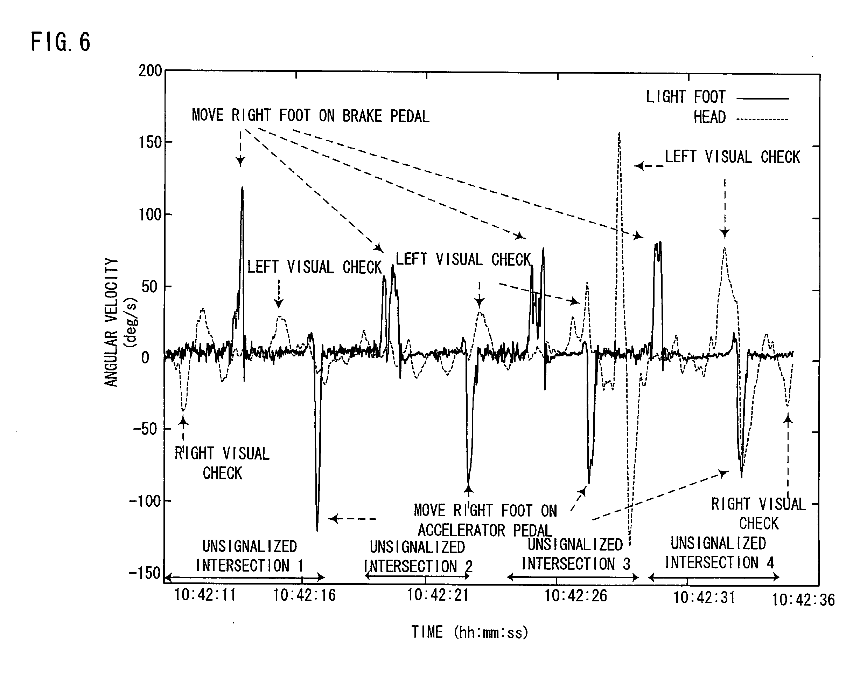

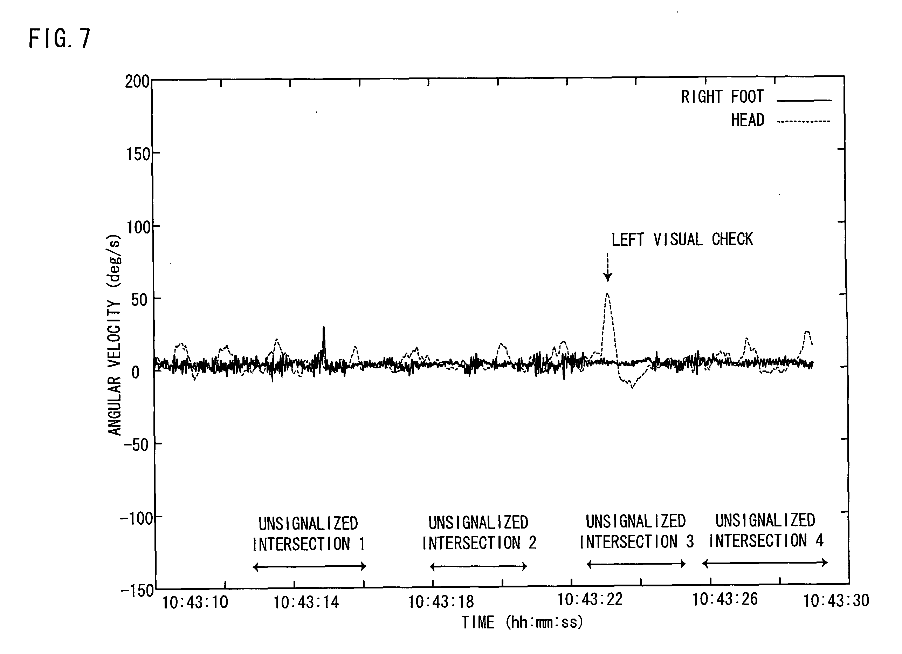

Inventors et al. carried out experiments by utilizing an actual motor vehicle at unsignalized intersections in order to survey how much detection of a right-and-left checking motion and a braking stance which is conventionally difficult to measure can be made by the angular velocity sensors 14 and 16. There are five test subjects. Each test subject is attached with the angular velocity sensors 14 and 16 at the head and the right toe as shown in Figure 2 and Figure 3 and drives in the order of 5 km. Figure 6 and Figure 7 show angular velocity data when a driver who is judged as a safe driver and a driver who is judged as an unsafe driver, by his or her passenger and a confirmation by the video image respectively drive a zone (both drivers drive the same zone) in which four successive unsignalized intersections exist. A horizontal axis shown in each of Figure 6 and Figure 7 is a time (hh:mm:ss, that is, hour: minute: second), a vertical axis is an angular velocity (deg/s), and solid lines indicate an output from the angular velocity sensor 16 attached to the right foot, and dashed lines indicate an output from the angular velocity sensor 14 attached to the head.

The angular velocity data of the test subject who is regarded as a safe driver shown in Figure 6 shows that he or she is sure to move the right foot to the brake pedal 32 (he or she does not necessarily foot down the pedal 32) and to frequently perform visual checks during passing through the intersection. The result in Figure 6 is roughly coincident with an occurring time and the number of occurrences of the right-and-left checking motion/the braking stance by the video checking.

On the other hand, the data of the test subject who is regarded as an unsafe driver in Figure 7 shows that he or she never moves the foot on the brake pedal 32 during passing through the intersection (never takes a braking stance) and never makes a preparation in case of emergency. If only a footing down force of the pedal is focused, it is unclear whether a braking stance is taken. However, by the angular velocity sensor 16, a movement of the right foot between the accelerator pedal 30 and the brake pedal 32 can be detected, and therefore, it is possible to determine whether or not a braking stance is taken.

As described above, by utilizing the angular velocity sensors 14 and 16, it becomes possible to measure a right-and-left checking motion and a braking stance by the driver without placing a burden on the driver.

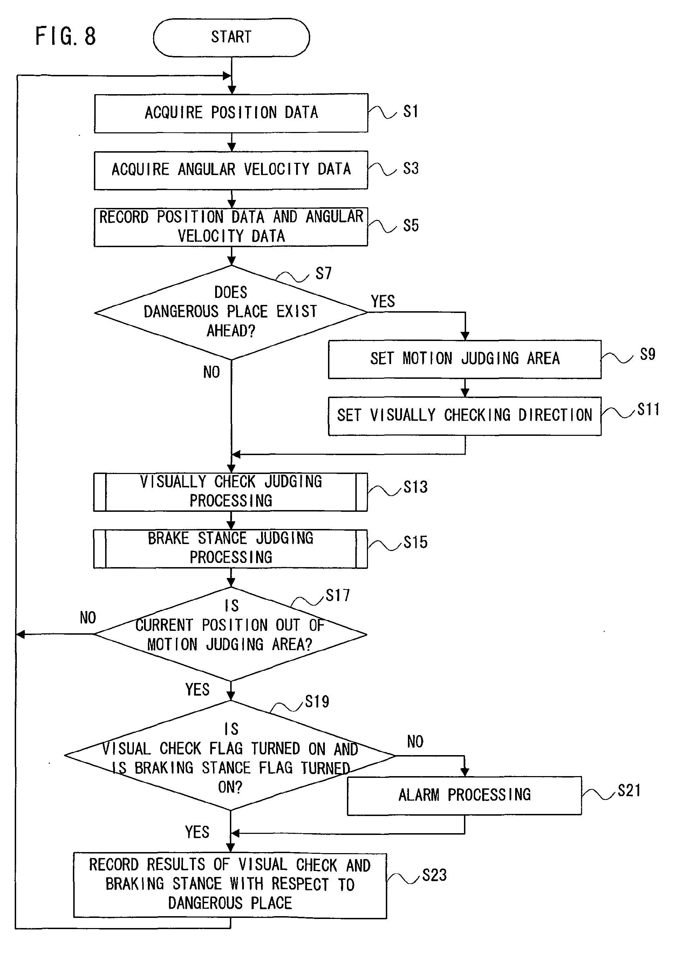

Next, referring to flowcharts, a specific operation of the system 10 is explained. Figure 8 shows one example of an operation of main processing of the computer 12 of the system 10. Here, Figure 8 shows a flowchart when a judgment as to a dangerous drive preventive intention is performed in real time.

In a step S1, position data indicating coordinates of a current position (latitude, longitude, altitude) is acquired from the position detecting device 18 and stored in the RAM. Next, in a step S3, angular velocity data detected by the angular velocity sensor 14 attached to the head and angular velocity data detected by the angular velocity sensor 16 attached to the right toe are acquired and stored in the RAM.

Then, the position data and the angular velocity data which are acquired are recorded in the driving data recording device 24 in a step S5. Here, the position data and angular velocity data are stored by being brought into correspondence with current time information acquired from a clock circuit integrated in the computer 12.

Successively, in a step S7, it is determined whether or not dangerous places exist ahead. For example, on the basis of the current position and the road data of the map data, a road (road ID) along which the driver currently drives is specified. As shown in Figure 5, as road data, coordinates of the starting point and the ending point and a width are registered, and from them, an area of each road can be calculated. Accordingly, by determining in which area the coordinates of the current position is included, it is possible to specify the current road. In addition, from the change of the current position, a traveling direction of the motor vehicle can be calculated. Thus, it is determined whether or not a dangerous place (coordinates of an accident-prone place) exists by an end point (starting point or ending point) of the current road in the travel direction. Furthermore, if the end point (starting point or ending point) of the current road in the travel direction is an intersection, with reference to the intersection data, whether or not an unsignalized intersection is determined and whether or not the accident-prone flag is turned on is determined.

Here, the judgment of the presence or absence of the dangerous place in the step S7 may be executed for each change of the road. Alternatively, when the driver approaches the end point of the current road a constant distance before, the judgment as to the presence or absence of the dangerous place may be executed on the road connected to the end point.

If "YES" in the step S7, that is, if dangerous places exist, a motion judging area of each dangerous place is set in a step S9. The visually checking motion and the braking stance need to be performed when the motor vehicle approaches the dangerous place, and therefore, when the motor vehicle approaches the dangerous place, whether or not a visually checking motion is performed and whether or not a braking stance is taken are judged. The motion judging area is an area for judging whether or not the motor vehicle approaches the dangerous place, and an area where a visually checking motion and a braking stance are to be detected. Thus, the motion judging area is set ahead of the dangerous place.

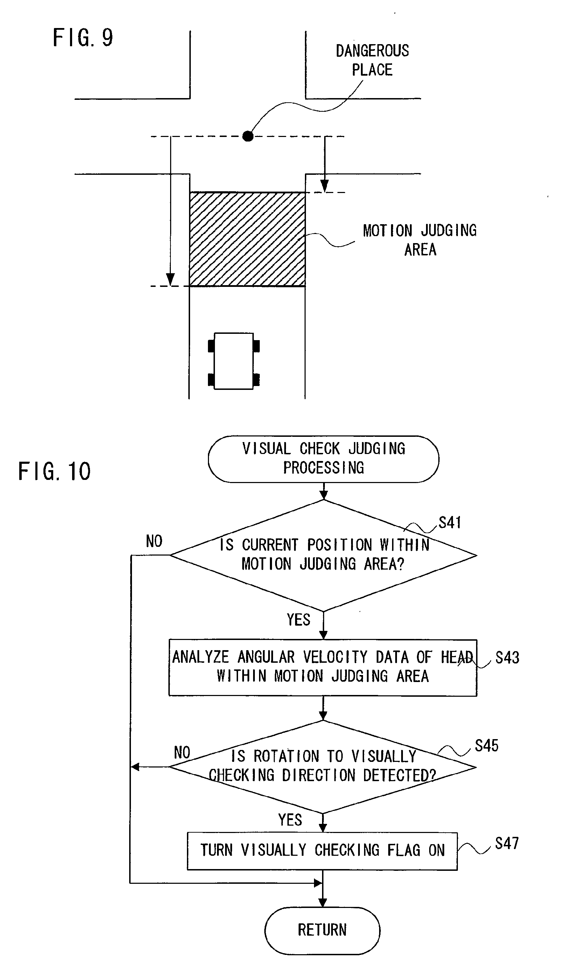

In this embodiment, as shown in Figure 9, a predetermined range ahead of the dangerous place is set as a motion judging area. Within the motion judging area, danger prevention or an averting motion, such as a visual check and braking stance need to be executed. For example, in a case that the dangerous place is an intersection, in view of the width of the road crossed at the intersection, a predetermined range may be set with the line of the entrance of the intersection centered. As one example, 2 or 3 m to 7 or 8 m ahead of the entrance may be set. Furthermore, in a case that the dangerous place is set not to an intersection but to a road, a predetermined range is set with reference to the dangerous place. Here, as an upper limit of the distance defining the motion judging area, an appropriate distance for which it is determined a visually checking motion and a braking stance (danger prevention or averting motion) may be started with respect to the dangerous place is selected. In addition, as a lower limit of the distance defining the motion judging area, an appropriate distance for which it is determined that a visually checking motion and a braking stance (danger prevention or averting motion) should be ended with respect to a dangerous place is selected.

Moreover, as a motion judging area, a different area may be set in correspondence with a velocity of the motor vehicle. More specifically, if it is determined that the velocity of the motor vehicle is high (more than a predetermined value), the motion judging area may be set at a place further away from the dangerous place whereas if it is determined that the velocity of the motor vehicle is low (equal to or less than a predetermined value), the motion judging area may be set at a place closer to the dangerous place. Here, the velocity of the motor vehicle is calculated from the change of the coordinates detected by the position detecting device 18 every predetermined time. Furthermore, in a case that the position detecting device 18 is provided with the speed sensor, the velocity detected by the sensor may be used.

Successively, in a step S11 in Figure 8, a visually checking direction of each dangerous place is set. For example, in a case that the dangerous place is an intersection, it is determined whether a road is connected to the left side or the right side of the intersection on the basis of the road data, and the direction that the road is connected is set as a visually checking direction. Accordingly, if the intersection is a crossroads as shown in Figure 9, the right and left directions are set. Furthermore, if the dangerous place is set not to an intersection but to a road, the right and left directions may be set, for example, or the left direction, the right direction, or both directions may be set in correspondence with a terrain, a feature, etc. of the dangerous place.

Alternatively, if "NO" in the step S7, that is, if a dangerous place does not exist ahead, the process proceeds to a step S13 as it is.

In the step S13, processing of judging whether a visual check as one of the danger preventing or averting motion is performed is executed. One example of an operation of the visual check judging processing is shown in Figure 10. In a step S41 shown in Figure 10, a current position is within the motion judging area. In a case that the motion judging area is set in correspondence with a distance from the dangerous place (or entrance of the intersection) as described above, a distance between the coordinates of each dangerous place and the coordinates of the current position is calculated, and it is determined whether or not each calculated distance is a value between the upper limit and the lower limit of the distance defining each motion judging area.

If "NO" in the step S41, the current position is not within the motion judging area, and therefore, the visual check judging processing is ended as it is, and the process returns to a step S15 in Figure 8. The visually checking motion with respect to the dangerous place needs to be performed within the motion judging area, and therefore, if the current position is not within the motion judging area, the analysis of the angular velocity data of the head is not performed.

On the other hand, if "YES" in the step S41, the analysis of the angular velocity data of the head detected after the current position enters the motion judging area is performed in a step S43. As shown in Figure 6, in a case that the driver performs a visually checking motion, the angular velocity to this direction is detected by the angular velocity sensor 14 attached to the head. Accordingly, by analyzing the angular velocity data after the current position enters the motion judging area, detection of a rotation to the set visually checking direction is tried.

Then, in a step S45, it is determined whether or not a rotation to the visually checking direction is detected, that is, it is determined whether or not a danger preventing motion or a danger averting motion is executed. For example, in a case that the visually checking direction is the left direction, it is determined whether or not a rotation to the left direction by more than a predetermined angle is detected. Furthermore, in a case that the visually checking direction is the right direction, it is determined whether or not a rotation to the right direction by more than a predetermined angle is detected. In addition, in a case that the visually checking direction is both directions, it is determined whether or not a rotation to the left direction by more than a predetermined angle and a rotation to the right direction by more than a predetermined angle are detected. Here, in a case of the visual check toward both directions, the head is rotated from a state that it is rotated to a first direction by more than a predetermined angle to a reverse direction, and thus, as a rotation to the reverse direction thereafter, a rotation from the first rotated state to the front and a rotation from the front to the reverse direction by more than a predetermined angle need to be detected.

If "YES" in the step S45, it is determined that a necessary visually checking motion (danger averting motion or danger preventing motion) is performed, and therefore, in a step S47, a visual check flag stored in a predetermined area of the RAM is turned on. On the other hand, if "NO" in the step S45, a necessary visually checking motion (danger averting motion or danger preventing motion) is not detected, and the visual check judging processing is ended, and the process returns to the step S15 in Figure 8.

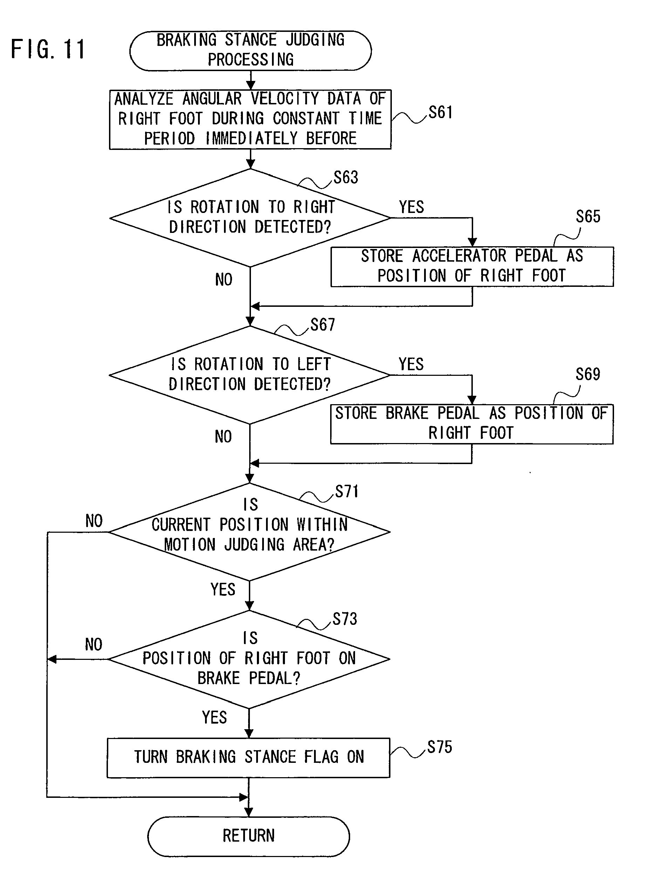

In the step S15 shown in Figure 8, braking stance judging processing being the other one of the danger averting motion or the danger preventing motion is executed. One example of an operation of the braking stance judging processing is shown in Figure 11. A motion of the braking stance corresponds to a situation that the right foot is on the side of the brake pedal 32 so as to immediately foot down the brake pedal 32 in case of emergency. Accordingly, in this embodiment, it is determined whether the position of the right foot is always on the side of the brake pedal 32 or on the side of the accelerator pedal 30. Then, when the current position is within the motion judging area, if the position of the right foot is on the side of the brake pedal 32, it is considered that a braking stance is taken, that is, the danger averting motion or the danger preventing motion can be performed.

In a step S61 in Figure 11, the angular velocity data of the right foot during a predetermined time period immediately before is analyzed. As shown in Figure 6, in a case that the driver moves the right foot between the brake pedal 32 and the accelerator pedal 30, an angular velocity to the left direction or the right direction is detected by the angular velocity sensor 16 attached to the right toe. Accordingly, the angular velocity data of the right foot during the predetermined time period immediately before is analyzed to try to detect a rotation in the right direction or the left direction.

Then, in a step S63, it is determined whether or not a rotation to the right direction is detected. For example, it is determined whether or not an angle of the rotation for which the sign is negative, and the absolute value is equal to or more than a predetermined value is detected. If "YES" is determined in the step S63, it is determined that the driver moves the light foot to the side of the accelerator pedal 30, and therefore, the accelerator is stored as a position of the right foot in a step S65. On the other hand, if "NO" in the step S63, the process proceeds to a step S67.

In the step S67, it is determined whether or not a rotation to the left direction is detected. For example, it is determined whether or not an angle of the rotation for which the sign is positive, and the absolute value is equal to or more than a predetermined value is detected. If "YES" in the step S67, it is determined that the driver moves the right foot to the side of the brake pedal 32, and therefore, in a step S69, the brake is stored as a position of the right foot. On the other hand, if "NO" in the step S67, the process proceeds to a step S71 as it is.

In the step S71, it is determined whether or not the current position is within the motion judging area. If "NO" in the step S71, the braking stance need not be judged, and therefore, the braking stance judging processing is ended as it is, and the process returns to the step S17 shown in Figure 8.

On the other hand, if "YES" in the step S71, that is, if it is determined that the motor vehicle approaches the dangerous place, it is judged whether or not a braking stance is taken. That is, in a step S73, it is determined whether or not the position of the right foot is on the brake pedal, that is, whether or not the danger preventing motion or the danger averting motion is executed. If "YES" in the step S73, it is determined that a braking stance (danger preventing motion or danger averting motion) is taken, and therefore, in a step S75, a braking stance flag stored in a predetermined area of the RAM is turned on. On the other hand, if "NO" in the step S73, a braking stance is not detected, and therefore, the braking stance judging processing is ended as it is, and the process returns to a step S17 shown in Figure 8.

In the step S17, it is determined whether or not the current position is out of the motion judging area. For example, it is determined whether or not the distance between the dangerous place (or the entrance of the intersection) and the current position is smaller than the lower limit of the distance defining the motion judging area. That is, in the step S17, it is determined whether or not a timing has come to finally judge whether or not a necessary motion is performed with respect to the dangerous place. If "NO" in the step S17, the process returns to the step S1.

On the other hand, if "YES" in the step S17, it is determined whether or not the visual check flag is turned on, and the braking stance flag is turned on in a step S19. If "NO" in the step S19, that is, if it is determined that the motor vehicle is going to arrive at the dangerous place without a necessary danger averting or a necessary prevention motion, alarm processing is executed in a step S21. Thus, the alarming device 22 outputs a warning sound, displays a warning image, and outputs vibrations.

On the other hand, if "YES" in the step S19, that is, if a necessary danger averting or a necessary prevention motion is performed, the process proceeds to a step S23 as it is. In the step S23, data indicating the result of the visual check and the braking stance with respect to the dangerous place are recorded in the driving data recording device 24. For example, information indicating whether or not a visual check is performed, information indicating whether or not a braking stance is taken, and time information, etc. are stored by being brought into correspondence with the coordinates of the dangerous place (or identifying information). After completion of the processing in the step S23, the process returns to the step S1.

According to this embodiment, by utilizing the angular velocity sensors 14 and 16, a visually checking motion and a braking stance are detected to thereby judge whether the danger averting or preventing motion is performed, and therefore, it is possible to successively check the dangerous drive preventive intention of the driver, output an alarm in real time, and record a judgment result of the dangerous drive preventive intention. In addition, the dangerous drive preventive intention of the driver can be quantitatively measured, and thus, it is of use for a preselection of a subject who has to take a reeducation course. Furthermore, a countermeasure to a loss of the durability of the effect of the reeducation course is made possible, such as evaluating the durability of the effect of the reeducation course, and making the subject take the course as necessary.

In addition, in the first embodiment, it is conceivable that any one of the step S21 and the step S23 in Figure 8 is omitted.

Here, in the above-described embodiment, the system 10 has the alarming device 22 and the driving data recording device 24, but in another embodiment, the system 10 may has any one of the alarming device 22 and the driving data recording device 24. In a case that only the alarming device 22 is included, the system 10 becomes an alarming system for issuing an alarm in real time depending on whether a motion for a dangerous driving prevention is performed whereas in a case that only the driving data recording device 24 is contained, the system 10 becomes a recording system for recording whether or not a motion for the dangerous driving prevention is performed.

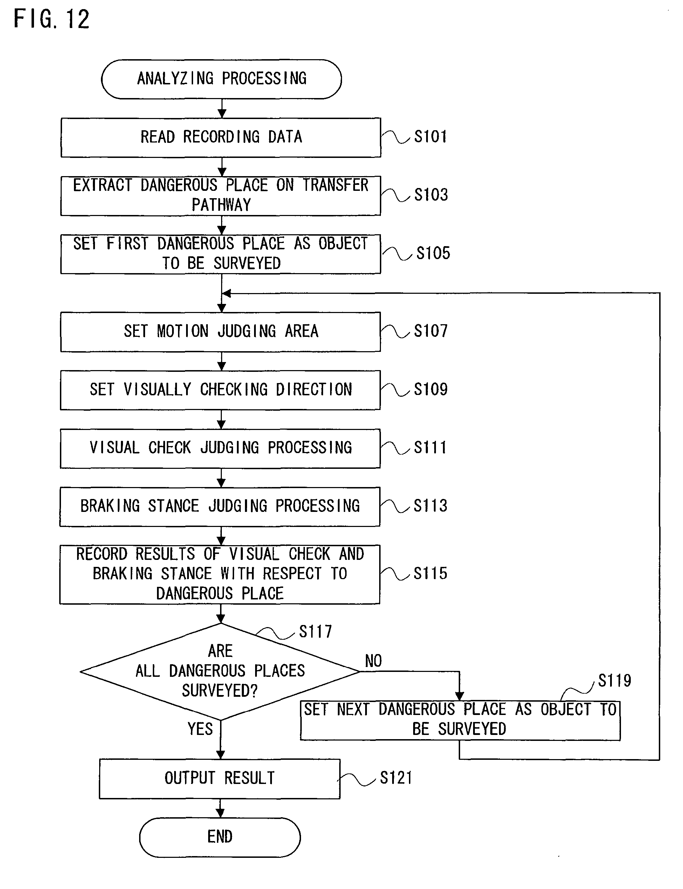

Furthermore, in each of the above-described embodiment, a visually checking motion and a braking stance are judged in real time, but in another embodiment, the data recorded during driving is analyzed in the post-mortem processing to thereby judge the danger averting or preventing motion, for example, a visually checking motion and a braking stance. In this case, processing similar to Figure 8 of successively reading recording data and making judgment may be executed postmortem, but another analyzing processing shown in Figure 12 may be executed.

### <Second Embodiment>

The post-mortem analyzing processing may be executed by the computer 12 of the system 10 in Figure 1. Alternatively, recording data (angular velocity data and position data) of the driving data recording device 24 is fetched and taken in another computer, and by this computer, the analyzing processing may be executed. It should be noted that the computer needs to store the map data (road data) of the map database 20, or the computer needs to be able to obtain the map data from the map database 20.

When the processing is started, in a step S101, recording data is first read onto the RAM. The recording data includes angular velocity data of the head, angular velocity data of the right toe, and position data of the motor vehicle which are recorded during driving. Here, the angular velocity data and the position data are detected at predetermined time intervals, and each data is time-series data.

Next, in a step S103, dangerous places on a transfer pathway are extracted. On the basis of the detected position data and the road data, a transfer pathway can be specified, and thus, the dangerous places on the transfer pathway can be extracted.

In a succeeding step S105, a first dangerous place on the pathway out of the extracted dangerous places is set as an object to be surveyed. It is determined whether or not a visually checking motion and a braking stance are performed on the set dangerous place, that is, whether or not a necessary danger averting motion or a necessary danger preventing motion is performed.

More specifically, in a step S107, a motion judging area of the dangerous place is set. The processing in the step S107 is the same as that in the step S9 in Figure 8 described above. In addition, in a step S109, a visually checking direction of the dangerous place is set. The processing in the step S109 is the same as that in the step S11 described above.

Then, in a step S111, visual check judging processing is executed with respect to the dangerous place. The visual check judging processing is approximately the same as the visual check judging processing in Figure 10, but this is not the real-time processing, and therefore, the judgment in the step S41 in Figure 10 is not required. More specifically, the angular velocity data of the head detected within the motion judging area is analyzed. Here, the angular velocity data is recorded by being associated with the position data by using the time information or without using it, and therefore, it is possible to extract only the angular velocity data detected within the motion judging area. If a rotation in a visually checking direction is detected by the analysis, it is considered that the visual check (danger averting or preventing motion) in this direction is performed, and the visual check flag is turned on.

In a succeeding step S113, braking stance judging processing with respect to the dangerous place is executed. More specifically, the angular velocity data of the right foot detected within the motion judging area is analyzed. Then, in a case that a predetermined rotation to the left direction is detected, it is considered that the position of the right foot is moved to the side of the brake, that is, that a danger averting or preventing motion is performed, and the braking stance flag is turned on. Here, even if the angular velocity data of the right foot is analyzed within the motion judging area, if no rotation in the right or left direction is detected, that is, if it is determined that the driver did not move the right foot in the motion judging area, processing of analyzing the angular velocity data of the right foot as far back as a certain period of time is repeated until a predetermined rotation to the right or left direction is detected to thereby specify the position of the right foot. In a case that the position of the right foot is on the side of the brake pedal, it is considered that a braking stance is taken, that is, that the danger averting or preventing motion is performed, and whereby, the braking stance flag is turned on.

Then, in a step S115, the judgment results of the visual check and the braking stance with respect to the dangerous place are recorded in the RAM. For example, information indicating whether or not a visual check with respect to the set direction is performed in correspondence with the coordinates (or identifying information) of the dangerous place is, and the information indicating whether or not a braking stance is taken are stored.

Successively, in a step S117, it is determined whether or not surveys on all the dangerous places have been performed, and if "NO", in a step S119, a next dangerous place is set to a subject to be surveyed. Then, the process returns to the step S107 to perform processing for judging a visually checking motion and a braking stance with respect to the dangerous places.

On the one hand, if "YES" in the step S117, the results are output in a step S121. For example, data including the judgment results with respect to all the dangerous places are generated and output as files, or the judgment results may be displayed on the display. In addition, on the basis of the judgment results, processing of evaluating the dangerous drive preventive intention by the driver is further executed to output the evaluation result. When the processing in the step S121 is ended, the analyzing processing is ended.

Furthermore, in the above-described first and second embodiments, the angular velocity sensors 14 and 16 are attached to the head and the right toe of the driver to record the angular velocity data by which a visually checking motion and a braking stance are judged. However, in another embodiment, the kind of necessary motions may be changeable. Specifically, attribute information of the dangerous place is stored in the map data, and depending on each dangerous place, only the visually checking motion may be judged, and only the braking stance may be judged. More specifically, at a railroad crossing, a stop sign, etc., for example, motor vehicles are suspended under the rule, and therefore, if the dangerous place is such places, only the visually checking motion may be judged without judging a braking stance. In addition, in another embodiment, the driver may be only one of the angular velocity sensor 14 of the head or the angular velocity sensor 16 of the right toe to thereby judge only one of the visually checking motion and the braking stance.

In addition, in the above-described first and second embodiments, if the position of the right foot is moved to the side of the brake pedal even once within the motion judging area, it is judged that the braking stance flag is turned on, that is, a braking stance is performed (see Figure 11). However, in another embodiment, if the position of the right foot is on the side of the brake pedal out of the motion judging area, the braking stance flag may be turned on. That is, a fact that the driver puts the right foot on the side of the brake pedal out of the motion judging area may be a condition for judgment of the braking stance. In addition, these conditions for judgment may be used differently depending on the attribute of the dangerous place (the degree of danger), for example. For example, with respect to a dangerous place with a high degree of danger, a fact that the right foot is moved to the side of the brake pedal into the dangerous place is a condition for taking a braking stance whereas with respect to a less dangerous place, a fact that the right foot is moved to the side of the brake pedal even once may be a condition for performing a braking stance.

### <Third Embodiment>

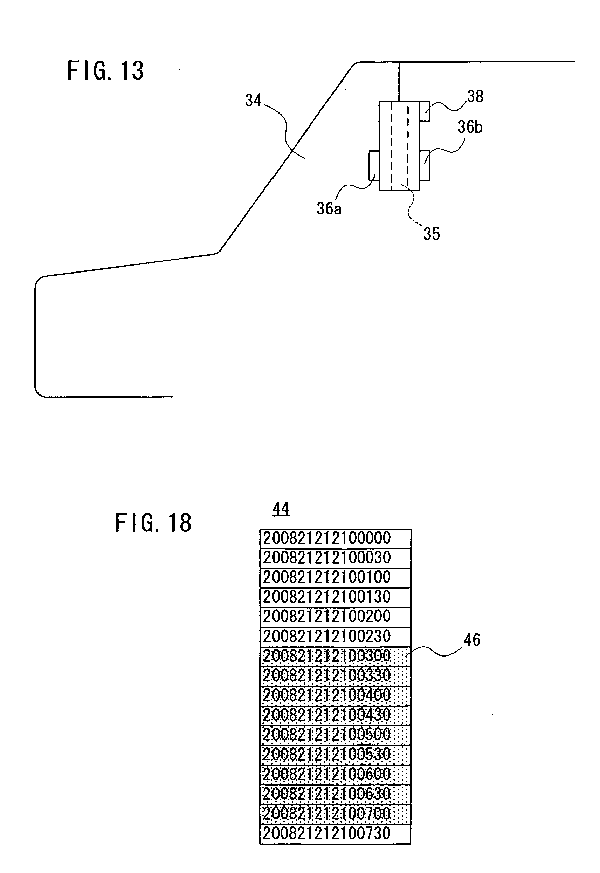

Figure 13 is an illustrative view showing one example of a state that a video camera and a microphone used in the third embodiment of the present invention are installed inside the motor vehicle. In this embodiment, in a case that a danger averting motion or a danger preventing motion is not executed, video image shooting an out-vehicle situation and an in-vehicle situation at least before and after the dangerous place are stored.

Here, the video image to be stored may be only the out-vehicle video image or only the in-vehicle video image. In addition, the video image may be a motion image in this embodiment, but may be a still image intermittently shot or successive still images.

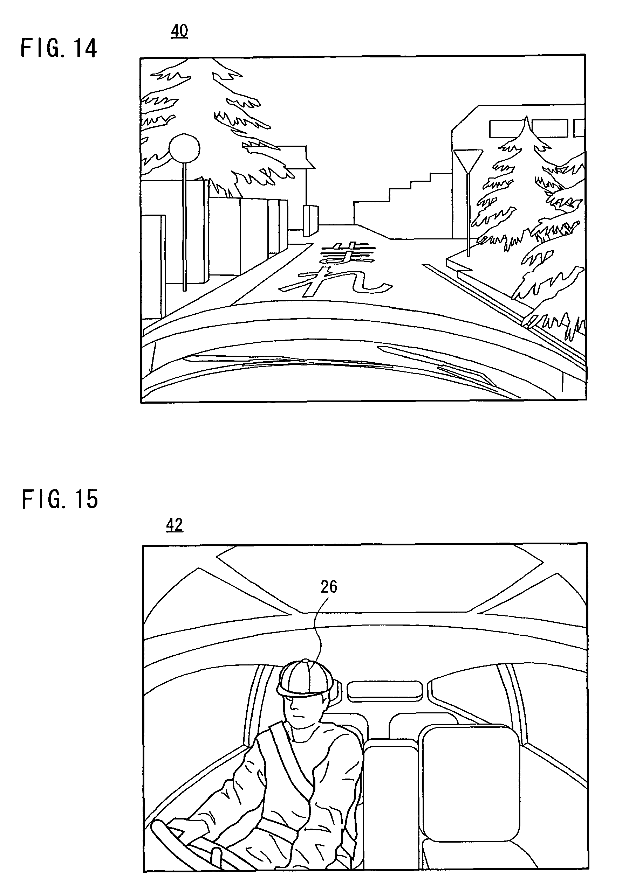

In the embodiment shown in Figure 13, video cameras 36a and 36b are installed at an appropriate place within the vehicle 34, for example, on a rear-view mirror 35 in order to shoot a video image. The video camera 36a is turned ahead of the vehicle 34, and thus shoots an out-vehicle video image 40 as shown in Figure 14. The video camera 36b turns backward of the vehicle 34 (in-vehicle), and thus shoots an in-vehicle video image 42 as shown in Figure 15. A driver in the in-vehicle video image 42 puts on the cap 26 as shown in Figure 2.

Here, angle of views or viewing angles of the video cameras 36a and 36b may be arbitrarily set, but in this embodiment, a camera having an angle of view of in the order of 140 degrees is used as an out-vehicle shooting camera 36a, and a camera having an angle of view in the order of 170 degrees is used as an in-vehicle shooting camera 36b.

Furthermore, for allowing shooting a video image during the night, these video cameras 36a and 36b may be an infrared camera or a camera which is used to be switchable between a function of a normal camera and a function of an infrared camera.

A microphone 38 is provided in addition to the video cameras 36a and 36b. The microphone 38 is mainly for capturing a sound in the vehicle and recording it. It is difficult to hear the sound of the out-vehicle from other sounds, and therefore, in this embodiment, a microphone for out-vehicle is not provided, but if necessary, another microphone (not illustrated) may be provided for recording an out-vehicle sound.

The installation location of the video cameras 36a and 36b and the microphone 38 may be any location if the locations are able to function for their intended purpose.

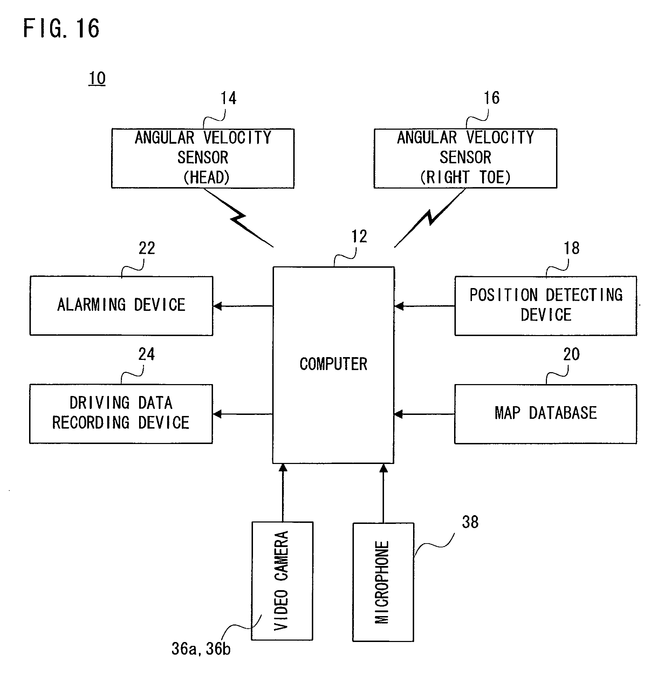

The configuration of the third embodiment is shown in Figure 16. The configuration shown in Figure 16 is the same as that shown in Figure 1 except for the following points.

In this embodiment, video images shot by the video cameras 36a and 36b are input to the computer 12 as video image data via a proper interface not shown. Furthermore, a sound captured by the microphone 38 is input to the computer 12 via a similar interface as sound data.

Then, in this embodiment, the driving data recording device 24 is set with a storing area or a recording area for storing the above-described video image data and sound data although especially not shown.

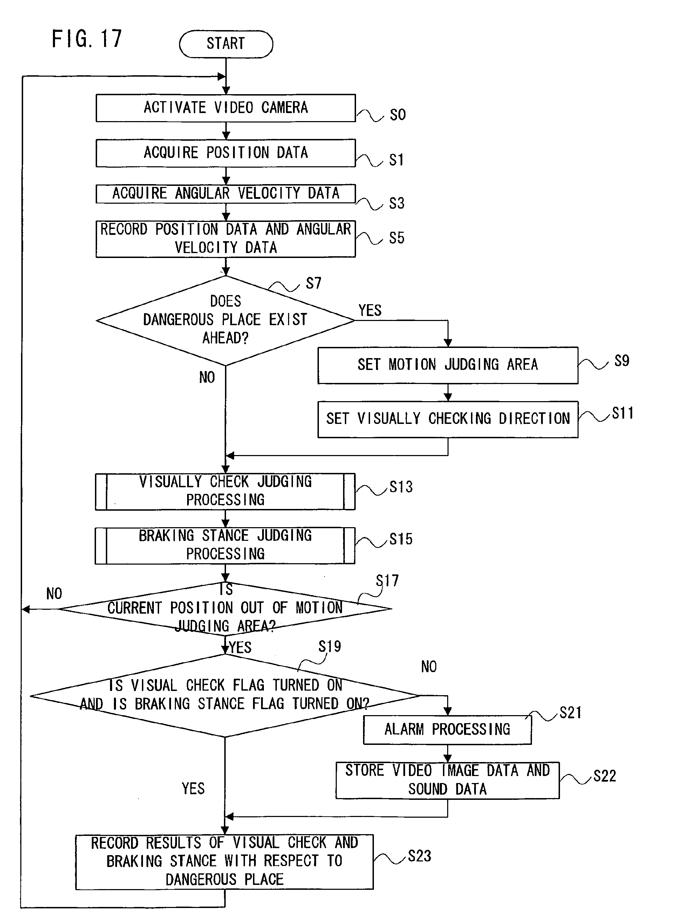

Referring to Figure 17, in a first step S0 of a dangerous drive preventive intention judging operation in the third embodiment, the computer 12 activates the video cameras 36a and 36b and the microphone 38 via an interface not shown. Accordingly, at a start of the dangerous drive preventive intention judging operation, the video cameras 36a, 36b and the microphone 38 are activated, so that the data of the out-vehicle video image 40 shown in Figure 14, the data of the in-vehicle video image 42 shown in Figure 15 and the sound data are recorded or stored at a first predetermined area of the driving data recording device 24 from this point.

Thereafter, the process proceeds to a step S1 to enable the computer 12 to acquire position data. Here, in this embodiment, steps S1 to S23 in Figure 17 are the same as the steps S1 to S23 in Figure 8 including the subroutines (Figure 10 and Figure 11) shown in the steps S13 and S15 except for that a step S22 is added, and therefore, the duplicated explanation is omitted.

As explained before, it is determined whether or not a visually checking motion is performed, that is, whether or not a danger averting or preventing motion is performed in the step S13, and whether or not a braking stance is taken, that is, whether or not dangerous driving averting or preventing motion is performed in the step S15. If a visually checking motion is performed, the visual check flag is turned on, and if not, the visual check flag is turned off. If a braking stance is performed, the braking stance flag is turned on, and if not, the braking stance flag is turned off.

Similar to the step S19 in Figure 8, in the step S19 in Figure 17, it is determined whether or not the visual check flag is turned on, and whether or not the braking stance flag is turned on. If "NO" in the step S19, that is, if it is determined that the motor vehicle is going to arrive at the dangerous place without a necessary danger averting or prevention motion, alarm processing is executed in the step S21. Thus, the alarming device 22 outputs a warning sound, displays a warning image, and outputs vibrations.

Then, in the step S22, the computer 12 stores the data of the out-vehicle video image and the data of the in-vehicle video image of the video cameras 36a and 36b. In this sense, the step S22 comprises a video image data storage (first video image data saver and second video image data saver) and sound data storage (first sound data saver and second sound data saver).

The computer 12 records or stores the data of the out-vehicle video image 40 shown in Figure 14 and the data of the in-vehicle video image 42 shown in Figure 15 from the video cameras 36a and 36b, and the sound data from the microphone 38 which are activated in the step S0 in a first predetermined area of the driving data recording device 24. Then, if it is found that a dangerous place exists ahead in the step S7, the motion judging area is set in the step S9. Accordingly, in the step S22, the data of the out-vehicle video image and the in-vehicle video image from the video cameras 36a and 36b and the sound data from the microphone 38 from when the vehicle passes through the motion judging area set in the step S9 to when "NO" is determined in the step S19 are recorded in a second predetermined area of the driving data recording device 24. The data of the out-vehicle video image, the data of the in-vehicle video image and the sound data which are recorded in the second predetermined area are not cleared, and thus stored in the second predetermined area of the data recording device 24 so long as they are erased in a predetermined method.

That is, in this embodiment, when the driver did not perform a danger averting motion or a danger preventing motion, the video images shot by the video cameras and the sound captured by the microphone until the motor vehicle passes through the motion judging area are stored as data. Accordingly, by reproducing the video image data and sound data and making the driver see and hear the video image data and the sound data thereafter, it is possible to clearly show that the danger averting or preventing motion was not performed.

Here, the video image data and the sound data stored in the step S22 are not restricted to data during which the motor vehicle passes through the motion judging area, and may be data defined by the time decided three minutes before and one minute after the processing in the step S22 is executed, for example.

Additionally, the video image data and the sound data may be stored in another format in the step S22.

As described before, in the first predetermined area of the driving data recording device 24, the video image data and the sound data which are acquired in the step S0 are successively recorded. At this time, as meta-information, position data of the vehicle acquired from the position detecting device 18 is recorded, and time data acquired from a clock circuit not shown is recorded as a time stamp. The time stamp is recorded at arbitrary time intervals, for example, 30 seconds. Such a time stamp 44 is shown in Figure 18. At the uppermost line in Figure 18, time data of "20081212100000" is shown, and this means February 12, 2008, 10: 00' 00", for example. In the next line, time data after a lapse of 30 seconds, "20081212100030", is recorded. Then, at the lowermost line, time data of "20081212100730" is recorded.

The aforementioned another format is adding the identifying symbol 46 shown by the shade shown in Figure 18 to the time data of the time stamp. In Figure 18, the identifying symbol 46 is added to the time data "20081212100300" to "20081212100700". Thus, with reference to the identifying symbol 46, it is possible to easily find a storing place within the driving data recording device 24 in which the video image data and the sound data are recorded when the danger averting or preventing motion was not performed. Then, if the video image data and the sound data stored in the driving data recording device 24 all the while are designated and recorded, it is possible to make the driver perceive a driving situation that a danger preventing or averting motion was not performed during the time period indicated between the two hours.

Additionally, in the third embodiment, by using the video cameras 36a and 36b, the out-vehicle video image and the in-vehicle video image are stored, but the video image data to be stored may be data of at least one of the out-vehicle video image and the in-vehicle video image.

Furthermore, the sound data is stored together with the video image data, but the sound data need not be stored. In this case, the microphone 38 can be omitted naturally.

In addition, it is considered that in the third embodiment, the step S21 and the step S23 shown in Figure 17 may be omitted. This is because it is possible to instill the driver with a danger preventing intention by merely storing the video image data when the danger preventing or averting motion was not performed.

In the third embodiment, if "NO" is determined in the step S19, that is, both of the visual check flag and the braking stance flag are turned off as a result of the processing in each of the steps S13 and S15, the processing in the step S22 is executed. However, as a modification of the third embodiment, if at least one of the visual check flag and the braking stance flag is turned off, the processing in the steps S21 and S22 may be executed. Alternatively, the processing in the step S21 is executed only when the two flags are turned off, and the processing in the step S22 may be executed even if only one of the two flags is turned off. On the contrary thereto, it may be possible to make a change such that the processing in the step S22 may be executed only when the two flags are turned off, and the processing in the step S21 may be executed even if one flag out of the two flags is turned off.

### <Fourth Embodiment>

The third embodiment described above is an embodiment in which when in the first embodiment, it is judged that the driver fails to perform a danger preventing motion or a danger averting motion, data of the in-vehicle and the out-vehicle video images shot by the video camera 36 and data of the in-vehicle and out-vehicle sounds captured by the microphone 38 are stored, and they are used for safe-driving education for not only the driver but also other drivers.

The same idea can be applied to the aforementioned second embodiment, and an embodiment configured in accordance therewith shall be a fourth embodiment. The configuration of the fourth embodiment is similar to that in Figure 16, and the operation is according to the flowchart shown in Figure 12. Thus, with the help of these drawings, the fourth embodiment is explained.

In the second embodiment, in the first step S101 in Figure 12, the processing of reading recording data is executed, but in the fourth embodiment, the recording data includes data of the in-vehicle and out-vehicle video images shot by the video camera 36 and data of the in-vehicle and out-vehicle sounds captured by the microphone 38 shown in Figure 16 are included. That is, in the fourth embodiment, by utilizing the configuration in Figure 16, the recording data is acquired. Accordingly, in this embodiment, the recording data recorded in the first predetermined area of the driving data recording device 24 includes the video image data and the sound data as well as angular velocity data and position data.

Then, visual check judging processing of judging whether or not a visual check as one of the danger averting or preventing motion is performed is executed in a step S111, and braking stance judging processing of judging whether or not a braking stance as one of the danger averting or preventing motion is taken is executed in a step S113. These processing in the steps S111 and S113 are shown in Figure 10 and Figure 11, and have already been explained.

Then, on the basis of these processing results, the results are recorded in the step S115, but similar to the step S22 (Figure 17) in the third embodiment, when as a result of the processing in each of the steps S111 and S113, at least any one of the visual check flag and the braking stance flag is not turned on, data of the in-vehicle and the out-vehicle video images shot by the video cameras 36a and 36b and sound data captured by the microphone 38 from when the vehicle passes through the motion judging area set in the step S107 (Figure 12) to when the determination results in the respective steps S111 and S113 are obtained may be stored in the second predetermined area of the driving data recording device 24, or the identifying symbol as shown in Figure 18 may be stored.

Moreover, in each of the above-described embodiments, in order to detect the movements of the head and the right toe of the driver, the angular velocity sensors 14 and 16 are utilized, but in another embodiment, another detecting device may be used. For example, it may be possible to use the acceleration sensor. In correspondence with a visually checking motion and a braking stance of the driver, an acceleration is changed, and therefore, by analyzing the acceleration data of the head and the acceleration data of the right toe as movement data, it is possible to judge whether or not a visually checking motion and a braking stance are performed.

The visually checking motion follows a movement in the right and left direction of the head, and therefore, the acceleration sensor of the head is attached so as to detect an acceleration in at least the right and the left direction of the head. In a case that a movement to the right or the left direction is detected on the basis of the acceleration data of the head, it is possible to judge that a visually checking motion in the left or the right direction is performed.

Furthermore, the braking stance can be judged by detecting a movement in the right and left direction of the right toe, and therefore, the acceleration sensor of the right toe is attached so as to detect an acceleration in at least the right and left direction of the right toe. In a case that a movement in the left direction is detected on the basis of the acceleration data of the right toe, it can be determined that the right foot is moved to the side of the brake pedal 32, that is, that a braking stance is taken. Additionally, in a case that a movement to the right direction is detected, it can be determined that the right foot is moved to the side of the accelerator pedal 30.

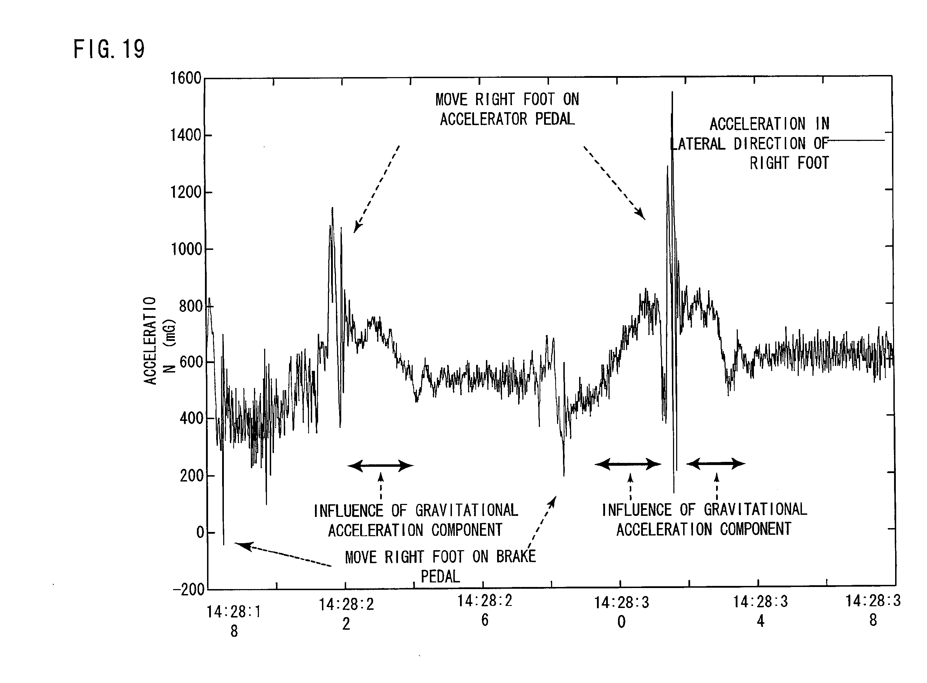

Here, the acceleration sensor is generally subjected to a gravitational acceleration, and thus, the output is an addition of a gravitational acceleration component corresponding to the inclination of the sensor and an actual acceleration component. In a case that an abrupt motion at emergency has to be measured, the influence of the gravitational acceleration component is less if any at all, but if a movement of the foot from one pedal to another has to be measured, the effect thereof is considerable. Figure 19 shows acceleration data when a movement of the right foot is measured by use of an acceleration sensor in place of the angular velocity sensor 16. The horizontal axis is a time, the vertical axis is an acceleration (mG), and an acceleration in a lateral direction (X-axis direction) is shown. This is experimental results executed on a course different from that in the experiments shown in Figure 6 and Figure 7 as described above. As shown in Figure 19, it can be found that the foot is moved to change the inclination of the sensor, and whereby, a gravitational acceleration component imparted in the X-axis direction is changed and mixed in the data as noise like a sine wave. When the right foot is moved from the accelerator pedal to the brake pedal, the inclination of the acceleration sensor of the right foot is changed, and therefore, it is desirable that a two-axis acceleration sensor or a three-axis acceleration sensor is applied as an acceleration sensor for detecting a movement of the right toe to thereby detect the movement of the right toe after removal of the noise due to the inclination of the sensor.

## Claims

1. A dangerous drive preventive intention judging system for judging a dangerous drive preventive intention of a driver of a motor vehicle with respect to a dangerous place, comprising:
a memory arranged to store angular velocity data detected and recorded by a head angular velocity sensor (14) attached to a head of said driver, angular velocity data detected and recorded by a toe angular velocity sensor (16) attached to the right toe of said driver, and position data of said motor vehicle detected and recorded by a position detecting device (18);
a first judger arranged to judge whether or not a visually checking motion is performed by said driver when a position of said motor vehicle approaches said dangerous place on the basis of the angular velocity data detected by said head angular velocity sensor and the position data of said motor vehicle; and
a second judger arranged to judge whether or not a braking stance is taken when a position of said motor vehicle approaches said dangerous place on the basis of the angular velocity data detected by said toe angular velocity sensor and position data of said motor vehicle, and
a result outputter arranged to output judgment results by said first judger and said second judger.

2. A dangerous drive preventive intention judging system according to claim 1, further comprising a first alarmer arranged to issue an alarm when said first judger judges that said visually checking motion was not performed.

3. A dangerous drive preventive intention judging system according to claim 2, further comprising a first video image data saver arranged to save video image data of at least one of an in-vehicle video image and an out-vehicle video image of said motor vehicle when said first judger judges that the visually checking motion was not performed.

4. A dangerous drive preventive intention judging system according to claim 2 or 3, further comprising a first sound data saver arranged to save sound data of at least an in-vehicle sound of said motor vehicle when said first judger judges that the visually checking motion was not performed.

5. A dangerous drive preventive intention judging system according to claim 1, further comprising a second alarmer arranged to issue an alarm when said second judger judges that a braking stance was not taken.

6. A dangerous drive preventive intention judging system according to claim 5, further comprising a second video image data saver arranged to save video image data of at least one of an in-vehicle video image and an out-vehicle video image of said motor vehicle when said second judger judges that the braking stance was not taken.

7. A dangerous drive preventive intention judging system according to claim 6, further comprising a second sound data saver arranged to save sound data of at least an in-vehicle sound of said motor vehicle when said second judger judges that the braking stance was not taken.

8. A dangerous drive preventive intention judging system according to claim 1, comprising a first recorder arranged to record a judgment result by said first judger.

9. A dangerous drive preventive intention judging system according to claim 1, further comprising a second recorder further arranged to record a judgment result by said second judger.

10. A dangerous drive preventive intention judging method for judging a dangerous drive preventive intention of a driver of a motor vehicle with respect to a dangerous place, including steps of:
(a) recording angular velocity data detected by a head angular velocity sensor (14) attached to a head of said driver;
(b) recording angular velocity data detected by a toe angular velocity sensor (16) attached to the right toe of said driver;
(c) recording position data of said motor vehicle, said position data detected by a position detecting device (18), and
(d) judging whether or not a visually checking motion by said driver is performed when said motor vehicle approaches said dangerous place by analyzing the angular velocity data detected by the head angular velocity sensor the angular velocity data detected by the toe angular velocity sensor, and the position data.

11. A dangerous drive preventive intention judging system according to claim 1, further comprising:
a map database (20) in which dangerous places are registered;
a determiner arranged to determine whether or not a dangerous place exists ahead on the basis of said map database and said position data;
a motion judging area setter arranged to set a motion judging area when said determiner determines that a dangerous place exists ahead, wherein
said first judger is arranged to judge whether or not a visually checking motion by said driver is performed when said motor vehicle exists within said motion judging area and wherein said second judger is arranged to judge whether or not a braking stance is taken when said motor vehicle is within said motion judging area.

12. A dangerous drive preventive intention judging system according to claim 1, further comprising:
a direction setter arranged to set a visually checking direction in which a visual check by said driver has to be performed, wherein said first judger is arranged to judge whether or not a rotation of said driver's head in said visually checking direction of said head is detected.

## Patentansprüche

1. System zum Beurteilen der Absicht eines präventiven gefährlichen Fahrmanövers zum Beurteilen der Absicht eines Fahrers eines Kraftfahrzeugs, ein präventives gefährliches Fahrmanövers an einer gefährliche Stelle durchzuführen, das Folgendes umfasst:
einen Speicher zum Speichern von Winkelgeschwindigkeitsdaten, die von einem am Kopf des genannten Fahrers angebrachten Kopf-Winkelgeschwindigkeitssensor (14) erkannt und aufgezeichnet werden, von Winkelgeschwindigkeitsdaten, die von einem am rechten Zeh des genannten Fahrers angebrachten ZehWinkelgeschwindigkeitssensor (16) erkannt und aufgezeichnet werden, und von von einem Positionserkennungsgerät (18) erkannten und aufgezeichneten Positionsdaten des genannten Kraftfahrzeugs;
eine erste Beurteilungsfunktion zum Beurteilen, auf der Basis der von dem genannten Kopf-Winkelgeschwindigkeitssensor erkannten Winkelgeschwindigkeitsdaten und der Positionsdaten des genannten Kraftfahrzeugs, ob eine Sichtprüfbewegung von dem genannten Fahrer durchgeführt wird oder nicht, wenn sich eine Position des genannten Kraftfahrzeugs der genannten gefährlichen Stelle nähert; und
eine zweite Beurteilungsfunktion zum Beurteilen, auf der Basis der von dem genannten Zeh-Winkelgeschwindigkeitssensor erkannten Winkelgeschwindigkeitsdaten und der Positionsdaten des genannten Kraftfahrzeugs, ob eine Bremshaltung eingenommen wird oder nicht, wenn sich eine Position des genannten Kraftfahrzeugs der genannten gefährlichen Stelle nähert, und
eine Ergebnisausgabefunktion zum Ausgeben von Beurteilungsergebnissen durch die genannte erste Beurteilungsfunktion und die genannte zweite Beurteilungsfunktion.

2. System zum Beurteilen einer Absicht eines präventiven gefährlichen Fahrmanövers nach Anspruch 1, das ferner eine erste Alarmierfunktion zum Ausgeben eines Alarms umfasst, wenn die genannte erste Beurteilungsfunktion urteilt, dass die genannte Sichtprüfbewegung nicht durchgeführt wurde.

3. System zum Beurteilen einer Absicht eines präventiven gefährlichen Fahrmanövers nach Anspruch 2, das ferner eine erste Videobilddaten-Speicherfunktion zum Speichern von Videobilddaten eines fahrzeuginternen Videobildes und/oder eines fahrzeugexternen Videobildes des genannten Kraftfahrzeugs umfasst, wenn die genannte erste Beurteilungsfunktion urteilt, dass die Sichtprüfbewegung nicht durchgeführt wurde.

4. System zum Beurteilen der Absicht eines präventiven gefährlichen Fahrmanövers nach Anspruch 2 oder 3, das ferner eine erste Tondatenspeicherfunktion zum Speichern von Tondaten von wenigstens einem fahrzeuginternen Ton des genannten Kraftfahrzeugs umfasst, wenn die genannte erste Beurteilungsfunktion urteilt, dass die Sichtprüfbewegung nicht durchgeführt wurde.

5. System zum Beurteilen der Absicht eines präventiven gefährlichen Fahrmanövers nach Anspruch 1, das ferner eine zweite Alarmierfunktion zum Ausgeben eines Alarms umfasst, wenn die genannte zweite Beurteilungsfunktion urteilt, dass keine Bremshaltung eingenommen wurde.

6. System zum Beurteilen der Absicht eines präventiven gefährlichen Fahrmanövers nach Anspruch 5, das ferner eine zweite Videobilddaten-Speicherfunktion zum Speichern von Videobilddaten eines fahrzeuginternen Videobildes und/oder eines fahrzeugexternen Videobildes des genannten Kraftfahrzeugs umfasst, wenn die genannte zweite Beurteilungsfunktion urteilt, dass die Bremshaltung nicht eingenommen wurde.

7. System zum Beurteilen der Absicht eines präventiven gefährlichen Fahrmanövers nach Anspruch 6, das ferner eine zweite Tondatenspeicherfunktion zum Speichern von Tondaten von wenigstens einem fahrzeuginternen Ton des genannten Kraftfahrzeugs umfasst, wenn die genannte zweite Beurteilungsfunktion urteilt, dass die Bremshaltung nicht eingenommen wurde.

8. System zum Beurteilen der Absicht eines präventiven gefährlichen Fahrmanövers nach Anspruch 1, das einen ersten Rekorder zum Aufzeichnen eines Beurteilungsergebnisses durch die genannte erste Beurteilungsfunktion umfasst.

9. System zum Beurteilen der Absicht eines präventiven gefährlichen Fahrmanövers nach Anspruch 1, das ferner einen zweiten Rekorder umfasst, der ferner zum Aufzeichnen eines Beurteilungsergebnisses durch die genannte zweite Beurteilungsfunktion ausgelegt ist.

10. Verfahren zum Beurteilen der Absicht eines präventiven gefährlichen Fahrmanövers zum Beurteilen der Absicht eines Fahrers eines Kraftfahrzeugs, ein gefährliches präventives Fahrmanövers an einer gefährlichen Stelle durchzuführen, das die folgenden Schritte beinhaltet:
(a) Aufzeichnen von Winkelgeschwindigkeitsdaten, die von einem am Kopf des genannten Fahrers angebrachten Kopf-Winkelgeschwindigkeitssensor (14) erkannt werden;
(b) Aufzeichnen von Winkelgeschwindigkeitsdaten, die von einem am rechten Zeh des genannten Fahrers angebrachten Zeh-Winkelgeschwindigkeitssensor (16) erkannt werden;
(c) Aufzeichnen von Positionsdaten des genannten Kraftfahrzeugs, wobie die genannten Positionsdaten von einer Positionserkennungsvorrichtung (18) erkannt werden; und
(d) Beurteilen, ob eine Sichtprüfbewegung von dem genannten Fahrer durchgeführt wird oder nicht, wenn sich das genannte Kraftfahrzeug der genannten gefährlichen Stelle nähert, durch Analysieren der vom Kopf-Winkelgeschwindigkeitssensor erkannten Winkelgeschwindigkeitsdaten, der vom Zeh-Winkelgeschwindigkeitssensor erkannten Winkelgeschwindigkeitsdaten und der Positionsdaten.

11. System zum Beurteilen der Absicht eines präventiven gefährlichen Fahrmanövers nach Anspruch 1, das ferner Folgendes umfasst:
eine Kartendatenbank (20), in der gefährliche Stellen registriert sind;
eine Ermittlungsfunktion zum Ermitteln, ob sich voraus eine gefährliche Stelle befindet oder nicht, auf der Basis der genannten Kartendatenbank und der genannten Positionsdaten;
eine Bewegungsbeurteilungsbereich-Einstellfunktion zum Einstellen eines Bewegungsbeurteilungsbereichs, wenn die genannte Ermittlungsfunktion feststellt, dass sich voraus eine gefährliche Stelle befindet, wobei
die genannte erste Beurteilungsfunktion so ausgelegt ist, dass sie beurteilt, ob eine Sichtprüfbewegung durch den genannten Fahrer durchgeführt wird oder nicht, wenn sich das genannte Kraftfahrzeug innerhalb des genannten Bewegungsbeurteilungsbereichs befindet, und wobei die genannte zweite Beurteilungsfunktion so ausgelegt ist, dass sie beurteilt, ob eine Bremshaltung eingenommen wird oder nicht, wenn sich das genannte Kraftfahrzeug innerhalb des genannten Bewegungsbeurteilungsbereichs befindet.

12. System zum Beurteilen der Absicht eines präventiven gefährlichen Fahrmanövers nach Anspruch 1, das ferner Folgendes umfasst:
eine Richtungseinstellfunktion zum Einstellen einer Sichtprüfrichtung, in der eine Sichtprüfung von dem genannten Fahrer durchgeführt werden soll, wobei
die genannte erste Beurteilungsfunktion so ausgelegt ist, dass sie beurteilt, ob eine Drehung des Kopfes des genannten Fahrers in der genannten Sichtprüfrichtung des genannten Kopfes erkannt wird oder nicht.

## Revendications

1. Système d'évaluation de l'intention d'éviter une conduite dangereuse, servant à évaluer l'intention du conducteur d'un véhicule à moteur d'éviter une conduite dangereuse en rapport avec un endroit dangereux, comprenant :
une mémoire adaptée pour stocker des données de vitesse angulaire détectées et enregistrées par un capteur de vitesse angulaire de la tête (14) fixé à la tête dudit conducteur, des données de vitesse angulaire détectées et enregistrées par un capteur de vitesse angulaire du bout du pied (16) fixé au bout du pied droit dudit conducteur, et des données de position dudit véhicule à moteur détectées et enregistrées par un dispositif détecteur de position (18) ;
un premier dispositif évaluateur adapté pour évaluer si oui ou non ledit conducteur effectue un mouvement de contrôle visuel lorsqu'une position dudit véhicule à moteur s'approche dudit endroit dangereux sur la base des données de vitesse angulaire détectées par ledit capteur de vitesse angulaire de la tête et des données de position dudit véhicule à moteur ; et
un deuxième dispositif évaluateur adapté pour évaluer si oui ou non une attitude de freinage est adoptée lorsqu'une position dudit véhicule à moteur s'approche dudit endroit dangereux sur la base des données de vitesse angulaire détectées par ledit capteur de vitesse angulaire du bout du pied et des données de position dudit véhicule à moteur, et
un dispositif de sortie de résultats adapté pour délivrer en sortie les résultats des évaluations effectuées par ledit premier dispositif évaluateur et ledit deuxième dispositif évaluateur.

2. Système d'évaluation de l'intention d'éviter une conduite dangereuse selon la revendication 1, comprenant en outre un premier dispositif d'alerte adapté pour émettre une alerte lorsque ledit premier dispositif évaluateur évalue que ledit mouvement de contrôle visuel n'a pas été effectué.

3. Système d'évaluation de l'intention d'éviter une conduite dangereuse selon la revendication 2, comprenant en outre un premier dispositif de sauvegarde de données d'images vidéo adapté pour sauvegarder des données d'images vidéo d'au moins une image vidéo intérieure au véhicule et une image vidéo extérieure au véhicule dudit véhicule à moteur lorsque ledit premier dispositif évaluateur évalue que le mouvement de contrôle visuel n'a pas été effectué.

4. Système d'évaluation de l'intention d'éviter une conduite dangereuse selon la revendication 2 ou la revendication 3, comprenant en outre un premier dispositif de sauvegarde de données audio adaptée pour sauvegarder des données audio d'au moins un son intérieur au véhicule dudit véhicule à moteur lorsque ledit premier dispositif évaluateur évalue que le mouvement de contrôle visuel n'a pas été effectué.

5. Système d'évaluation de l'intention d'éviter une conduite dangereuse selon la revendication 1, comprenant en outre un deuxième dispositif d'alerte adapté pour émettre une alerte lorsque ledit deuxième dispositif évaluateur évalue qu'une attitude de freinage n'a pas été adoptée.

6. Système d'évaluation de l'intention d'éviter une conduite dangereuse selon la revendication 5, comprenant en outre un deuxième dispositif de sauvegarde de données d'images vidéo adapté pour sauvegarder des données d'images vidéo d'au moins une image vidéo intérieure au véhicule et une image vidéo extérieure au véhicule dudit véhicule à moteur lorsque ledit deuxième dispositif évaluateur évalue que l'attitude de freinage n'a pas été adoptée.

7. Système d'évaluation de l'intention d'éviter une conduite dangereuse selon la revendication 6, comprenant en outre un deuxième dispositif de sauvegarde de données audio adapté pour sauvegarder des données audio d'au moins un son intérieur au véhicule dudit véhicule à moteur lorsque ledit deuxième dispositif évaluateur évalue que l'attitude de freinage n'a pas été adoptée.

8. Système d'évaluation de l'intention d'éviter une conduite dangereuse selon la revendication 1, comprenant un premier dispositif d'enregistrement adapté pour enregistrer un résultat d'une évaluation effectuée par ledit premier dispositif évaluateur.

9. Système d'évaluation de l'intention d'éviter une conduite dangereuse selon la revendication 1, comprenant en outre un deuxième dispositif d'enregistrement adapté également pour enregistrer un résultat d'une évaluation effectuée par ledit deuxième dispositif évaluateur.

10. Procédé d'évaluation de l'intention d'éviter une conduite dangereuse, servant à évaluer l'intention du conducteur d'un véhicule à moteur d'éviter une conduite dangereuse en rapport avec un endroit dangereux, comprenant les étapes consistant à :
(a) enregistrer des données de vitesse angulaire détectées par un capteur de vitesse angulaire de la tête (14) fixé à la tête dudit conducteur ;
(b) enregistrer des données de vitesse angulaire détectées par un capteur de vitesse angulaire du bout du pied (16) fixé au bout du pied droit dudit conducteur ;
(c) enregistrer des données de position dudit véhicule à moteur, lesdites données de position étant détectées par un dispositif détecteur de position (18) ; et
(d) évaluer si oui ou non ledit conducteur effectue un mouvement de contrôle visuel lorsque ledit véhicule à moteur s'approche dudit endroit dangereux en analysant les données de vitesse angulaire détectées par le capteur de vitesse angulaire de la tête, les données de vitesse angulaire détectées par le capteur de vitesse angulaire du bout du pied et les données de position.

11. Système d'évaluation de l'intention d'éviter une conduite dangereuse selon la revendication 1, comprenant en outre :
une base de données cartographiques (20) dans laquelle des endroits dangereux sont enregistrés ;
un dispositif de détermination adapté pour déterminer s'il existe ou non un endroit dangereux à l'avant sur la base de ladite base de données cartographiques et desdits données de position ;
un dispositif de définition de zone d'évaluation de mouvement adapté pour définir une zone d'évaluation de mouvement lorsque ledit dispositif de détermination détermine qu'il existe un endroit dangereux à l'avant,
dans lequel ledit premier dispositif évaluateur est adapté pour évaluer si oui ou non ledit conducteur effectue un mouvement de contrôle visuel lorsque ledit véhicule à moteur se trouve à l'intérieur de ladite zone d'évaluation de mouvement et dans lequel ledit deuxième dispositif évaluateur est adapté pour évaluer si oui ou non une attitude de freinage est adoptée lorsque ledit véhicule à moteur se trouve dans ladite zone d'évaluation de mouvement.

12. Système d'évaluation de l'intention d'éviter une conduite dangereuse selon la revendication 1, comprenant en outre :
un dispositif de définition de direction adapté pour définir une direction de contrôle visuel dans laquelle un contrôle visuel doit être effectué par ledit conducteur,
dans lequel ledit premier dispositif évaluateur est adapté pour évaluer si oui ou non une rotation de la tête dudit conducteur dans ladite direction de contrôle visuel dudit tête est détectée.
